# FASCICULE DE BREVET EUROPEEN

(11) **EP 3 099 182 B1**
(45) Date de publication et mention de la délivrance du brevet: **16.09.2020**
(21) Numéro de dépôt: 15701766.6
(22) Date de dépôt: 27.01.2015
(51) Int. Cl.: A23K 10/12, A23K 10/30, A23K 10/37, A23K 20/189, A23K 50/10, A47B 95/00, F16B 12/20, A61P 3/00, C12N 9/42, C12N 9/24

(54) **UTILISATION D'UNE COMPOSITION ENZYMATIQUE DANS L'ALIMENTATION DES RUMINANTS**
VERWENDUNG EINER ENZYMMISCHUNG ZUR FÜTTERUNG VON WIEDERKÄUERN
USE OF AN ENZYMATIC COMPOSITION IN THE FEED OF RUMINANTS

(30) Priorité: 27.01.2014 FR 1450650
(43) Date de publication de la demande: 07.12.2016
(73) Titulaire: Etablissements J. Soufflet, 10400 Nogent Sur Seine (FR)
(72) Inventeur: DELORD, Benoît, F-63122 Ceyrat (FR); TOURNAT, Mathieu, F-56000 Vannes (FR)
(74) Mandataire: Lavoix
(86) Numéro de dépôt international: PCT/EP2015/051621
(87) Numéro de publication internationale: WO 2015/110663

(56) Documents cités:
- US-A- 5 720 971
- US-A1- 2004 126 459
- US-A1- 2008 220 125
- US-A1- 2012 028 332
- P YU ET AL: "Improving the nutritional value of oat hulls for ruminant animals with pretreatment of a multienzyme cocktail: In vitro studies", JOURNAL OF ANIMAL SCIENCE, vol. 83, no. 5, 1 mai 2005 (2005-05-01), pages 1133-1141, XP055147986,

## Description

La présente invention concerne l'amélioration des performances zootechniques des ruminants par ajout d'additifs alimentaires non-médicamenteux dans l'alimentation.

Le progrès génétique chez les ruminants a permis d'obtenir des animaux d'élevage caractérisés par des performances zootechniques très élevées. Ceci se traduit par des niveaux de production laitière très hauts (plus de 40 kg/vache/jour) et des gains de poids très élevés chez les bovins à l'engrais (plus de 1500 g/animal/jour). Ces performances nécessitent une ingestion plus importante d'aliments par les animaux et une alimentation très riche afin de couvrir leurs besoins d'entretien et de production.

L'augmentation du niveau d'ingestion et l'augmentation de la part de concentré dans la ration des ruminants ont pour effets de diminuer l'efficacité métabolique des animaux. Les animaux utilisent moins bien les nutriments car le transit est accéléré et est donc moins efficace. Il existe donc un besoin important de trouver des solutions nutritionnelles capables d'améliorer le rendement de transformation de la ration (fourrages et concentrés) en produits (lait ou viande).

Il est connu que ce qui limite la digestibilité globale de la ration des ruminants est la digestibilité des fibres, cellulose et hémicellulose, alors que l'utilisation d'enzymes dans l'alimentation des ruminants est décrite dans l'art antérieur (Beauchemin et al. (2003) J. Anim. Sci. E.Suppl.2:E37-E47). Il a par ailleurs été proposé d'utiliser des amylases bactériennes (US 2009/0324571) ou des amylases fongiques (WO 03/068256) afin d'augmenter la digestibilité de l'amidon chez les ruminants. Néanmoins, les auteurs reconnaissent d'une part la variabilité importante des réponses des animaux à la supplémentation en enzymes et donc le besoin de trouver des additifs spécifiques pour les ruminants, et d'autre part le besoin de trouver des additifs visant l'amélioration de la digestibilité des polysaccharides majoritaires dans l'alimentation des ruminants : la cellulose et l'hémicellulose. Il a ainsi été proposé d'ajouter à l'alimentation des ruminants des complexes enzymatiques purifiés incluant des activités cellulase, xylanase, β-glucanase, pectinase, mannanase, et α-galactosidase afin d'augmenter la digestibilité des fourrages ingérés (WO 2009/0006362). Toutefois, ces additifs ne sont utilisés qu'avec des fourrages et ne sont pas utilisés avec des aliments concentrés (céréales, coproduits, tourteaux) alors que l'ajout d'aliments concentrés est le principal moyen utilisé pour améliorer les performances zootechniques des ruminants.

Il existe donc un besoin important de nouveaux additifs pour l'alimentation des ruminants permettant d'améliorer les performances zootechniques des animaux, même nourris avec une alimentation optimisée comprenant par exemple des aliments concentrés.

Il a été proposé d'utiliser différents types de microorganismes producteurs d'enzymes afin d'améliorer les performances zootechniques des ruminants (Beauchemin et al. (2004) Can. J. Anim Sci. 84:23-36). Une fois la fermentation menant à la production de ces enzymes terminée, les enzymes sont généralement séparées des résidus de fermentation et des microorganismes producteurs. Toutefois, les types et activités des enzymes produites varient largement selon le type de microorganisme utilisé, le substrat utilisé et les conditions de culture employées, ce qui mène à une forte hétérogénéité dans les effets observés chez les animaux et limite donc leur intérêt pour l'exploitant. En particulier, l'utilisation d'une combinaison efficace d'activités enzymatiques est difficile à déterminer, surtout avec des enzymes produites par des organismes génétiquement modifiés en fermentation en milieu liquide. Le mode d'administration des enzymes est également problématique lorsque l'on utilise des enzymes sous forme liquide, car celles-ci sont rapidement lessivées et inactivées dans le rumen.

La demande US 2012/028332 décrit une composition enzymatique, obtenue par fermentation submergée d'une matière végétale comprenant des pectines et des arabinanes par *Aspergillus tubingensis.*

L'article Yu et al. (2005) J. Animal Sci. 83 :1133-1141 décrit l'utilisation d'un cocktail multienzymatique comprenant des activités acide férulique estérase, xylanase, cellulase, endo-glucanase et β-glucanase, pour améliorer la dégradation de la matière sèche de coques d'avoine ou de paille de blé.

Le brevet US 5720971 décrit un complément enzymatique comprenant des activités cellulasique et xylanasique dans un ratio spécifique et l'utilisation de ce complément enzymatique pour traiter des fourrages de légumineuses ou des céréales fourragères.

La présente invention résulte de la découverte inattendue par les inventeurs qu'il est possible d'obtenir, de manière reproductible, une composition multienzymatique présentant des activités enzymatiques cellulasiques, xylanasiques et β-glucanasiques à des niveaux donnés, par fermentation en milieu solide de céréales et/ou de coproduits de céréales et/ou de coproduits d'oléagineux par un champignon filamenteux de type *Aspergillus* section *Nigri* du sous-clade *Aspergillus tubingensis,* et que cette composition enzymatique permet, lorsqu'elle est apportée dans la ration alimentaire d'un ruminant, quel qu'il soit, d'améliorer ses performances zootechniques, en particulier d'améliorer la production laitière, l'efficacité alimentaire, l'indice de consommation, la prise de poids, d'augmenter l'activité de rumination, d'augmenter la capacité d'ingestion des fourrages, d'augmenter la digestibilité des fourrages et/ou des concentrés et de réduire la production de méthane.

La présente invention est définie par les revendications.

La présente divulgation divulgue une composition enzymatique comprenant un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, fermenté avec une souche d'*Aspergillus* section *Nigri,* plus particulièrement d'*Aspergillus tubingensis,* ladite composition enzymatique présentant:
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition.

La présente invention concerne ainsi une composition enzymatique comprenant un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, fermenté avec une souche d'*Aspergillus* section *Nigri* du sous-clade *Aspergillus tubingensis* par fermentation en milieu solide, ladite composition enzymatique présentant:
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition.

Elle a également pour objet un procédé de fabrication de cette composition enzymatique comprenant une étape de fermentation en milieu solide d'un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, avec une souche du sous-clade *A. tubingensis,* plus particulièrement avec une souche d'*Aspergillus tubingensis* ou d'*Aspergillus neoniger,* de manière davantage préférée avec une souche d'*Aspergillus tubingensis,* au moins jusqu'à ce que le produit de fermentation présente les valeurs minimales suivantes d'activité enzymatique :
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition, la fermentation étant de préférence arrêtée à l'apparition des premières spores dans le milieu de culture.

Elle concerne également un additif pour l'alimentation des ruminants qui comprend cette composition enzymatique.

La présente invention a également pour objet l'utilisation de cet additif comme ingrédient, en particulier comme ingrédient non médicamenteux, de compositions alimentaires ou de prémix pour l'alimentation des ruminants.

Elle porte aussi sur un prémix pour l'alimentation des ruminants comprenant cet additif, ainsi que sur une composition alimentaire supplémentée pour ruminants comprenant une quantité efficace de cet additif ou de ce prémix, en association avec des aliments appropriés pour les ruminants.

Un autre objet de la présente invention concerne un procédé de fabrication d'une composition alimentaire supplémentée pour ruminants comprenant le mélange de l'additif tel que défini ci-dessus ou du prémix tel que défini ci-dessus avec des aliments appropriés pour les ruminants.

La présente invention concerne également l'utilisation non-thérapeutique de la composition enzymatique telle que définie ci-dessus, de l'additif tel que défini ci-dessus, du prémix tel que défini ci-dessus ou de la composition alimentaire telle que définie ci-dessus, pour améliorer les performances zootechniques d'un ruminant.

Elle vise enfin un procédé non-thérapeutique d'amélioration des performances zootechniques d'un ruminant dans lequel on fait ingérer au ruminant une quantité efficace de la composition enzymatique telle que définie ci-dessus, de l'additif tel que défini ci-dessus, du prémix tel que défini ci-dessus ou de la composition alimentaire telle que définie ci-dessus.

### Description détaillée de l'invention

### Ruminants

Dans le contexte de l'invention, le terme "ruminants" désigne tout mammifère herbivore polygastrique dont la digestion a totalement ou partiellement lieu au travers d'un processus de remastication de l'alimentation après son ingestion.

Le tube digestif des ruminants est composé de 4 compartiments : le rumen, le réticulum, l'omasum et l'abomasum. Parmi ces 4 compartiments, le rumen est le plus important car la plupart des nutriments y sont digérés. La digestion y correspond à un processus de fermentation impliquant une flore microbienne importante et variée responsable de la cellulolyse, de l'hémicellulolyse, de la protéolyse, de la production d'acide, de méthane, de la synthèse de vitamines, etc. De par le rôle primordial de la flore ruminale, la physiologie digestive des ruminants est donc considérablement différente de celle des animaux monogastriques, pour qui la flore joue un rôle secondaire.

De tels animaux sont bien connus de l'homme du métier et incluent par exemple les bovins, les ovins, les caprins, les cervidés et les camélidés. De préférence, dans le contexte de l'invention, le ruminant est un bovin.

Par "bovin" ou "boviné", on entend ici une sous-famille des bovidés comprenant plusieurs espèces importantes d'animaux d'élevage. Les bovins incluent en particulier la vache, en particulier la vache laitière, la vache allaitante, la génisse, le veau, le broutard, le taurillon, le bœuf, le bœuf à l'engrais, le taureau, le buffle, le yack, le gayal et le banteng. De préférence, quand le ruminant utilisé dans le cadre de l'invention est un bovin, il est choisi parmi la vache, en particulier la vache laitière, le veau, le broutard, le veau sous la mère, le bœuf et le bœuf à l'engrais.

Par "ovin", on entend ici les herbivores ruminants du genre *Ovis.* Les ovins incluent en particulier le mouflon, le mouton, la brebis, la vacive et l'agneau.

Par "caprin", on entend ici les herbivores ruminants du genre *Capra.* Les caprins incluent en particulier la chèvre, le bouc, le chevreau et le bouquetin.

Par "cervidé", on entend ici les ruminants de la famille des *Cervidae,* portant des bois. Les cervidés incluent en particulier, le cerf, le hère, le daguet, la biche, le faon, le chevreuil, le brocard, la chevrette, le renne, le daim, la daine et l'élan.

Par "camélidé", on entend ici des mammifères artiodactyles de la famille des *Camelidae.* Les camélidés incluent en particulier le dromadaire, le chameau, la chamelle, le lama et l'alpaga.

De préférence, dans le contexte de l'invention, le ruminant est un animal d'élevage.

### Composition enzymatique

La composition enzymatique décrite ici comprend, consiste essentiellement en, ou consiste en un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, fermenté avec une souche d'*Aspergillus* section *Nigri,* de préférence une souche d'*Aspergillus tubingensis,* ladite composition enzymatique présentant :
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition.

Les activités enzymatiques ci-dessus sont exprimées par gramme de composition séchée.

Par "xylanase", on entend ici une enzyme de la classe EC 3.2.1.8 de la nomenclature des enzymes de l'Union Internationale de Biochimie et de Biologie Moléculaire (IUBMB), qui dégrade le polysaccharide linéaire β-1,4-xylane en xylose, dégradant ainsi en particulier l'hémicellulose.

Les techniques de mesure de l'activité xylanasique sont bien connues de l'homme du métier. Typiquement, pour mettre en évidence cette activité, on peut faire agir la solution enzymatique sur une solution de xylane d'avoine soluble lié à un chromophore, le Rémazol Brillant Blue R, et mesurer les oligomères libérés par l'action de la solution enzymatique. Ceux-ci sont retrouvés dans la fraction soluble après précipitation à l'éthanol. Le milieu réactionnel préférablement utilisé dans cette méthode est composé de 130 µL de solution d'azo-xylane d'avoine (MEGAZYME) 10 g/L, 50 µL de solution enzymatique diluée dans du tampon acétate 0,4 M, pH 4,70. On effectue de préférence la réaction à 31°C pendant 20 minutes. La réaction est typiquement arrêtée par ajout de 500 µL d'éthanol à 96%. La densité optique du surnageant obtenu après centrifugation (10 minutes à 3000 tours/minute à 20°C) est lue à 590 nm. Une unité d'activité xylanase (AXC) peut alors être définie comme la quantité d'enzyme qui, diluée à raison de 1 unité/mL, à pH 4,70 et à 30°C, libère, à partir d'une solution de Rémazol Brillant Blue R xylane, des oligomères non précipitables dans l'éthanol tels que la densité optique du surnageant soit de 0,93 à 590 nm.

De préférence, la composition enzymatique selon l'invention présente une activité xylanase supérieure à 500 AXC par gramme de composition, de manière davantage préférée une activité xylanase supérieure à 750 AXC par gramme de composition, plus préférablement une activité xylanase supérieure à 1000 AXC par gramme de composition, de manière préférée entre toutes une activité xylanase supérieure ou égale à 2000 AXC par gramme de composition.

Par "β-glucanase", on entend ici une enzyme de la classe EC 3.2.1.6 de la nomenclature des enzymes de l'Union Internationale de Biochimie et de Biologie Moléculaire (IUBMB), qui clive le glucane. Les β-glucanases incluent en particulier la β-1,3-glucanase qui clive les β-1,3-glucanes, tels que le callose ou le curdlan, la β-1,6-glucanase qui clive les β-1,6-glucanes, la cellulase, l'endo-β-1,4-glucanase spécifique du xyloglucane ou l'exo-β-1,4-glucanase spécifique du xyloglucane.

Les techniques de mesure de l'activité β-glucanase sont bien connues de l'homme du métier. Typiquement, pour mettre en évidence cette activité, on peut faire agir la solution enzymatique sur une solution de β-glucane d'orge lié à un chromophore, le Rémazol Brillant Blue R. L'hydrolyse, à pH 4,8 de ce substrat par les activités β-glucanases libère des oligomères liés au chromophore et non précipitables par de l'éthanol dont la concentration est évaluée par mesure de l'absorbance à 590 nm. Le milieu réactionnel préférablement utilisé dans cette méthode est composé de 130 µL de solution d'azo-barley glucan (MEGAZYME) dilué au 4/5^{ème} dans un tampon de dilution acétate phosphate 0,1 M et ajusté à pH 4,75, 50 µL de solution enzymatique diluée dans du tampon acétate phosphate 0,1 M, pH 4,60. On effectue de préférence la réaction à 31°C pendant 20 minutes. La réaction est typiquement arrêtée par ajout de 620 µL de solution de précipitation (30 g d'acétate de sodium trihydraté et 3 g d'acétate de zinc dans 1 litre d'éthanol à 96%). La densité optique du surnageant obtenu après centrifugation (10 minutes à 3000 tours/minute à 20°C) est lue à 590 nm. Une unité d'activité β-glucanase (BGU) peut être alors définie comme la quantité d'enzyme qui, diluée à une concentration de 1 unité par mL dans les conditions de dosage (30°C et pH 4,8), libère des oligomères du β-glucane d'orge lié au Rémazol Brillant Blue R non précipitables dans l'éthanol, de telle façon que l'absorbance du surnageant est de 0,9 à 590 nm.

De préférence, la composition enzymatique selon l'invention présente une activité β -glucanase supérieure à 500 BGU par gramme de composition, de manière davantage préférée une activité β-glucanase supérieure à 600 BGU par gramme de composition, plus préférablement une activité β-glucanase supérieure à 1000 BGU par gramme de composition, de manière préférée entre toutes une activité β-glucanase supérieure ou égale à 1500 BGU par gramme de composition.

Par "cellulase", on entend ici une enzyme de la classe EC 3.2.1.4 de la nomenclature des enzymes de l'Union Internationale de Biochimie et de Biologie Moléculaire (IUBMB), qui clive la cellulose, la lichenine et les β-glucanes de céréales, polymère de glucoses liés en β1-4. Les "cellulases" incluent en particulier l'endo-1,4-β-D-glucanase, la β-1,4-glucanase, la β-1,4-endoglucane hydrolase, la cellulase A, la cellulosine AP, l'endoglucanase D, l'alkali cellulase, la cellulase A 3, la celludextrinase, la 9.5 cellulase, l'avicelase, la pancellase SS et la 1,4-(1,3,1,4)-β-D-glucane 4-glucanohydrolase.

Les techniques de mesure de l'activité cellulase sont bien connues de l'homme du métier. Typiquement, pour mettre en évidence cette activité, on peut faire agir la solution enzymatique sur une solution de carboxyméthyl cellulose partiellement dépolymérisée et liée à un chromophore, le Rémazol Brillant Blue R. L'hydrolyse, à pH 4,5 de ce substrat par les activités cellulases libère des oligomères liés au chromophore et non précipitables par de l'éthanol dont la concentration est évaluée par mesure de l'absorbance à 590 nm. Le milieu réactionnel préférablement utilisé dans cette méthode est composé de 100 µL de solution d'azo-carboxyméthyl-cellulose (Mégazyme 90504a) 20 g/L et pH 4,5, 100 µL de solution enzymatique diluée dans du tampon acétate 0,1 M, pH 4,60. On effectue de préférence la réaction à 41°C pendant 10 minutes. La réaction est typiquement arrêtée par ajout de 500 µL de solution de précipitation (40 g d'acétate de sodium trihydraté et 4 g d'acétate de zinc dans 1 litre d'éthanol à 96%). La densité optique du surnageant obtenu après centrifugation (10 minutes à 3000 tours/minute à 20°C) est lue à 590 nm. Une unité d'activité carboxyméthylcellulase (CMC) peut être alors définie comme la quantité d'enzyme qui, diluée à une concentration de 1 unité par mL dans les conditions de dosage (41°C et pH4,5), libère des oligomères de la carboxyméthyl cellulose partiellement dépolymérisée liée au Rémazol Brillant Blue R non précipitables par la soultion de précipitation, de telle façon que l'absorbance du surnageant est de 1,0 à 590 nm.

De préférence, la composition enzymatique selon l'invention présente une activité cellulase supérieure à 50 CMC par gramme de composition, de manière davantage préférée une activité cellulase supérieure à 75 CMC par gramme de composition, plus préférablement une activité cellulase supérieure à 120 CMC par gramme de composition, de manière préférée entre toutes, une activité cellulase supérieure ou égale à 180 CMC par gramme de composition.

Dans un mode de réalisation particulièrement préférée, la composition enzymatique selon l'invention présente une activité xylanase supérieure ou égale à 2000 AXC par gramme de composition, une activité β-glucanase supérieure ou égale à 1500 BGU par gramme de composition et une activité cellulase supérieure ou égale à 180 CMC par gramme de composition.

La composition enzymatique selon l'invention peut en outre comprendre des activités enzymatiques autres que les activités xylanase, β-glucanase et cellulase ci-dessus, mais à des niveaux très faibles. De préférence, la composition enzymatique selon l'invention est dépourvue d'activité pectinase, mannanase, amylase et/ou α-galactosidase. De préférence, la composition enzymatique selon l'invention est dépourvue d'activité acide férulique estérase.

Par "composition dépourvue d'activité enzymatique X", on entend ici une composition dans laquelle l'activité enzymatique X n'est pas détectable par les techniques conventionnelles de détection d'activité enzymatique. Autrement dit, une composition dépourvue d'activité enzymatique X ne comprend pas de protéine ayant une activité enzymatique X ou comprend des protéines ayant une activité enzymatique X dans une concentration (par exemple déterminée par des techniques protéomiques) tellement faible que l'activité enzymatique X correspondante ne peut pas être mesurée par les techniques conventionnelles de détection d'activité enzymatique.

Les activités xylanase, β-glucanase et cellulase ci-dessus proviennent de la fermentation du substrat, en particulier du substrat principal, compris dans la composition enzymatique selon l'invention, avec une souche d'*Aspergillus* section *Nigri* du sous-clade *Aspergillus tubingensis,* plus particulièrement une souche d'*Aspergillus tubingensis.*

Par "substrat principal", on entend ici le substrat qui représente au moins 70%, de préférence au moins 75%, au moins 80%, au moins 85%, au moins 90%, au moins 95% ou 100%, du substrat utilisé lors de la fermentation avec une souche d'*Aspergillus* section *Nigri* du sous-clade *Aspergillus tubingensis,* plus particulièrement une souche d'*Aspergillus tubingensis.*

Ce substrat, en particulier ce substrat principal, est sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza. De préférence, le substrat est sélectionné dans le groupe constitué d'un son de blé, d'un tourteau de colza, et d'un mélange de ceux-ci. De manière préférée entre toutes, le substrat, en particulier le substrat principal, est un mélange de son de blé et de tourteau de colza. Le son de blé et le tourteau de colza peuvent être présents dans ce mélange dans des proportions massiques allant de 10/90 (autrement dit 10% de son de blé pour 90% de tourteau de colza) à 90/10 (autrement dit 90% de son de blé pour 10% de tourteau de colza), de préférence de 15/85 (autrement dit 15% de son de blé pour 85% de tourteau de colza) à 80/20 (autrement dit 80% de son de blé pour 20% de tourteau de colza), de préférence de 20/80 (autrement dit 20% de son de blé pour 80% de tourteau de colza) à 70/30 (autrement dit 70% de son de blé pour 30% de tourteau de colza), de préférence de 25/75 (autrement dit 25% de son de blé pour 75% de tourteau de colza) à 60/40 (autrement dit 60% de son de blé pour 40% de tourteau de colza), de manière davantage préférée de 20/80 (autrement dit 20% de son de blé pour 80% de tourteau de colza) à 50/50 (autrement dit 50% de son de blé pour 50% de tourteau de colza), de manière encore préférée de 30/70 (autrement dit 30% de son de blé pour 70% de tourteau de colza) à 50/50 (autrement dit 50% de son de blé pour 50% de tourteau de colza).

Le substrat utilisé lors de la fermentation avec une souche d'*Aspergillus* section *Nigri* du sous-clade *Aspergillus tubingensis,* plus particulièrement une souche d'*Aspergillus tubingensis,* peut comprendre en outre à titre de substrat complémentaire :
- des tourteaux d'oléagineux, tels que des tourteaux de soja, tourteaux de germes de maïs, tourteaux de tournesol, tourteaux de palmiste, tourteaux de lin, tourteaux d'arachide, tourteaux de cacao, tourteaux de coprah, tourteaux de coton, tourteaux de pépins de raisins, grignons d'olive, tourteaux de caméline, tourteaux de jatropha ou tourteaux de sésame ;
- des coproduits céréaliers autres que le son de blé, tels que farine, remoulage, remoulage bis, son, gluten feed, gluten meal, drêche de distillerie/éthanolerie, drêche et soluble de distillerie/éthanolerie, drêche de brasserie, épi égrené, radicelle, germe ou brisure, d'avoine, de blé, d'orge, de triticale, de riz, de seigle ou de maïs ;
- des sources d'azote organiques telles que des acides aminés, concentrés de protéine de soja, concentrés de protéine de pomme de terre, gluten de blé ou gluten de maïs ;
- des sources d'azote inorganiques telles que de l'urée, l'ammoniac, des sels d'ammonium ou des vinasses ;
- des sels minéraux de sodium, potassium, chlore, calcium ou phosphore ; et/ou
- des oligo-éléments tels que du fer, cuivre, zinc, manganèse, cobalt, iode ou sélénium.

De préférence, le substrat utilisé lors de la fermentation avec une souche d'*Aspergillus* section *Nigri* du sous-clade *Aspergillus tubingensis,* plus particulièrement une souche d'*Aspergillus tubingensis,* peut comprendre en outre à titre de substrat complémentaire des tourteaux d'oléagineux, en particulier des tourteaux de germes de maïs.

Le substrat tel que défini ci-dessus est fermenté avec une souche d'*Aspergillus* section *Nigri* du sous-clade *Aspergillus tubingensis,* plus préférablement une souche d'*Aspergillus tubingensis* ou d'*Aspergillus neoniger,* plus préférablement une souche d'*Aspergillus tubingensis,* afin d'obtenir les activités xylanase, β-glucanase et cellulase telles que définies ci-dessus. Dans un mode de réalisation alternatif, le substrat tel que défini ci-dessus est fermenté avec une souche d'*Aspergillus* section *Nigri* du sous-clade *Aspergillus tubingensis* qui n'est pas une souche d'*Aspergillus tubingensis.* La fermentation est une fermentation en milieu solide.

Dans le contexte de l'invention, la "fermentation en milieu solide" est définie comme la culture de microorganismes sur des supports solides humides, sur des supports inertes ou sur des substrats insolubles qui peuvent être utilisés comme source de carbone et d'énergie. Le processus de fermentation a lieu en absence ou quasi-absence d'eau libre dans l'espace entre les particules de substrat. Au contraire, la "fermentation submergée" fait ici référence à une culture de microorganismes dans laquelle, à la fois les nutriments et les microorganismes sont submergés dans un milieu aqueux.

De préférence, le substrat tel que défini ci-dessus est fermenté par fermentation en milieu solide avec une souche d'*Aspergillus tubingensis.*

Par *"Aspergillus* section *Nigri"* on entend ici une section du genre *Aspergillus* anciennement appelée groupe *A. niger,* qui comprend typiquement 26 espèces telles que décrites dans Varga et al. (2011) Studies in Mycology 69:1-17, regroupées en 5 clades principaux : le clade *A. niger,* qui comprend les espèces *A. neoniger, A. costaricaensis, A. vadensis, A. eucalypticola, A. piperis, A acidus, A. tubingensis, A. awamori, A. niger* et *A. brasiliensis* ; le clade *A. carbonarius,* qui comprend les espèces *A. ibericus, A. sclerotiicarbonarius, A carbonarius* et *A. sclerotioniger* ; le clade *A. heteromorphus,* qui comprend les espèces *A. ellipticus* et *A. heteromorphus* ; le clade *A. homomorphus,* qui comprend l'espèce *A. homomorphus* ; et le clade *A. aculeatus,* qui comprend les espèces *A. fijiensis, A. aculeatus, A. aculeatinus, A. uvarum, A. indologenus, A. japonicus* et *A. violaceofuscus.* De préférence, la souche d'*Aspergillus* section *Nigri* décrite ici est une souche du clade *A. niger.* Dans un exemple décrit ici, la souche d'*Aspergillus* section *Nigri* décrite ici est une souche du clade *A. niger* qui n'est pas une souche d'A. *tubingensis.*

Comme indiqué ci-dessus, le clade *A. niger* comprend 10 espèces regroupées en 3 sous-clades : le sous-clade *A. tubingensis,* qui comprend les espèces *A. neoniger, A. costaricaensis, A. vadensis, A. eucalypticola, A. piperis, A acidus* et *A. tubingensis* ; le sous-clade *A. niger,* qui comprend les espèces *A. awamori* et *A. niger* ; et le sous-clade *A. brasiliensis* qui comprend l'espèce *A. brasiliensis.* La souche d'*Aspergillus* section *Nigri* utilisée dans le cadre de l'invention est une souche du sous-clade *A. tubingensis.* Dans un mode de réalisation particulier de l'invention, la souche d'*Aspergillus* section *Nigri* utilisée dans le cadre de l'invention est une souche du sous-clade *A. tubingensis* qui n'est pas une souche d'*A. tubingensis.* De manière davantage préférée, la souche d'*Aspergillus* section *Nigri* utilisée dans le cadre de l'invention est une souche d'*Aspergillus tubingensis* ou d'*Aspergillus neoniger,* de manière encore préférée une souche d'*Aspergillus tubingensis.*

Par *"Aspergillus tubingensis",* on entend ici un membre des Aspergillus section nigri, dont la souche caractéristique est la souche *Aspergillus tubingensis* Mosseray décrite dans La Cellule (1934) 43:245-247. C'est un champignon filamenteux produisant des conidies noires, ubiquitaire du sol, saprophyte et capable de se développer sur de nombreux substrats naturels complexes. *Aspergillus tubingensis* n'apparait pas dans la liste des agents pathogènes de l'annexe III de la Directive 2000/54/EC concernant la protection des travailleurs contre les risques liés à des agents biologiques au travail.

Des exemples de souches d'*Aspergillus tubingensis* sont bien connues de l'homme du métier et incluent *Aspergillus tubingensis* Mosseray ATCC MYA-81, *Aspergillus tubingensis* Mosseray ATCC MYA-83, *Aspergillus tubingensis* Mosseray ATCC MYA-84, *Aspergillus tubingensis* Mosseray ATCC MYA-4879, *Aspergillus tubingensis* Mosseray ATCC MYA-77, *Aspergillus tubingensis* Mosseray ATCC MYA-78, *Aspergillus tubingensis* Mosseray ATCC MYA-79, *Aspergillus tubingensis* Mosseray ATCC MYA-82, *Aspergillus tubingensis* Mosseray ATCC MYA-80, *Aspergillus tubingensis* Mosseray ATCC 10550, *Aspergillus tubingensis* Mosseray ATCC 76608 et *Aspergillus tubingensis* Mosseray ATCC 201255.

Par *"Aspergillus neoniger",* on entend ici un membre des Aspergillus section nigri, dont la souche caractéristique est la souche *Aspergillus neoniger* Varga décrite dans Varga et al. (2011) Studies in Micology 69:1-17.

Des exemples de souches *d'Aspergillus neoniger* sont bien connues de l'homme du métier et incluent *Aspergillus neoniger* CBS 115656 ou NRRL 62634 et *Aspergillus neoniger* CBS 115657.

De préférence, le substrat tel que défini ci-dessus est pré-traité avant fermentation en vue de le pasteuriser ou le stériliser. Le traitement thermique peut consister en un chauffage par exemple dans un autoclave. Le substrat peut ainsi être autoclavé pendant 15 à 45 min, de préférence pendant 20 à 40 min, de manière davantage préférée pendant 35 min, à une température comprise entre 90 et 125°C, de préférence entre 95 et 115°C, de manière davantage préférée à une température de 105°C.

De manière particulièrement préférée, le substrat tel que défini ci-dessus est pré-humidifié de manière à atteindre une matière sèche de 40 à 100%, de préférence une matière sèche de 45 à 90%, de préférence une matière sèche de 50 à 80%, de préférence une matière sèche de 55 à 70%, de manière préférée à 60% de matière sèche.

Avantageusement, on peut régler le pH lors de l'humidification dans la gamme de 4,8 à 6,2, de préférence de 5,0 à 6,0, de préférence de 5,2 à 5,8, de manière préférée entre toutes à 5,6, afin d'améliorer l'effet pasteurisant du traitement thermique et le démarrage de la fermentation souhaitée.

L'inoculation du substrat tel que défini ci-dessus peut se pratiquer avec tout inoculum approprié. L'homme du métier connaît de multiples façons de préparer un inoculum convenable à partir d'une souche d'*Aspergillus tubingensis* sélectionnée. La dose d'inoculation est avantageusement d'au moins 1×10⁷ spores par gramme de matière sèche initiale de substrat.

La teneur en eau du substrat au début de la fermentation est de préférence réglée entre 40 et 50%, de préférence à 45% de la masse totale du substrat et de l'eau et on la maintient de préférence sensiblement dans cet intervalle pendant la fermentation, par exemple en procédant périodiquement à des apports d'eau pour compenser la perte en eau du milieu. L'expression "sensiblement maintenue" signifie qu'il est tolérable que le taux d'humidité prenne une valeur s'écartant de 5 unités % de l'intervalle 40-50% pendant une relativement brève période entre deux ajustements successifs du taux d'humidité ou en fin de fermentation. Le taux d'humidité du substrat peut en effet avoir tendance à baisser au cours de la fermentation par évaporation sous l'effet de l'augmentation de température générée par la croissance fongique.

La fermentation peut être conduite dans tout réacteur approprié. Des exemples de réacteur utilisable sont ceux décrits dans l'article de A. Durand et al. publié dans Agro-Food-Industry Hi-Tech (Mai-Juin 1997, pages 39-42).

La fermentation peut être conduite pendant une période de 1 à 3 jours, de préférence de 30 à 60 heures, de manière particulièrement préférée pendant 48 heures. De préférence, la fermentation est arrêtée à l'apparition des premières spores dans le milieu de culture, la présence de spores pouvant gêner les animaux lors de l'ingestion du fait de leur caractère volatil.

La température du milieu est de préférence maintenue entre 28 et 38°C, de préférence entre 30 et 36°C, de manière davantage préférée à 33°C.

De préférence, la fermentation est réalisée en conditions aérobies et de préférence à l'obscurité.

Le produit de fermentation ainsi obtenu est un produit solide humide. Il peut être séché ou déshydraté, de préférence à une température modérée, par exemple inférieure à 45°C, pour ne pas affecter l'activité enzymatique. Le séchage ou la déshydratation peut être effectué par toute technique appropriée bien connue de l'homme du métier, telle que l'utilisation d'un lit fluidisé, la lyophilisation, l'étuvage, l'étuvage à vide ou la zéodratation.

Il peut également être congelé, de préférence à l'état humide, à basse température, par exemple à -20°C.

Le produit de fermentation peut également être extrait dans l'eau et récupéré sous forme liquide par toute technique appropriée bien connue de l'homme du métier. L'extraction du produit de fermentation peut être mise en œuvre par toute technique bien connue de l'homme du métier, en particulier par extraction en solution aqueuse, extraction en solution alcoolique, extraction par solvants, homogénéisation haute pression, extraction supercritique, extraction par lits fluidisés, broyage, broyage cryogénique, décompression, cavitation, bullage, extraction par ultrasons, adsorption sur résines ou zéolites. La ou les enzyme(s) sous forme liquide peu(ven)t être ensuite purifiée(s) par toute technique bien connue de l'homme du métier, en particulier par centrifugation, filtration, ultrafiltration, chromatographie, utilisation de membranes ou précipitation.

La composition enzymatique selon l'invention peut être sous toute forme appropriée pour son utilisation dans un additif. Elle est de préférence sous forme brute, non extraite.

Par "forme brute", on entend ici le milieu de fermentation contenant les activités enzymatiques telles que définies ci-dessus, le substrat fermenté et le champignon *Aspergillus* section *Nigri,* en particulier le champignon *Aspergillus tubingensis* tel que défini ci-dessus. Le milieu de fermentation après fermentation du substrat par une souche d'*Aspergillus* section *Nigri,* plus particulièrement une souche d'*Aspergillus tubingensis,* peut en particulier être déshydraté et/ou broyé avant d'être utilisé directement dans la composition enzymatique selon l'invention. Ce broyage peut être effectué par toute technique appropriée bien connue de l'homme du métier, telle que la micronisation, le broyage par cisaillement, le broyage par impact, le cryobroyage ou l'émiettage.

La composition enzymatique selon l'invention peut en outre être standardisée. Une telle standardisation permet avantageusement d'assurer une bonne homogénéité de la composition et de faciliter son utilisation lors de l'étape de fabrication des aliments.

### Additif pour l'alimentation des ruminants

La présente invention concerne un additif, de préférence non-médicamenteux, pour l'alimentation des ruminants, tels que définis dans la section *"Ruminants"* ci-dessus, qui comprend, consiste essentiellement en, ou consiste en une composition enzymatique telle que définie dans la section *"Composition enzymatique"* ci-dessus.

Par "additif", on entend ici un composant ou mélange de composants qui peut être ajouté à un aliment, à une ration alimentaire ou à un régime alimentaire d'un animal, ou donné à ingérer à l'animal.

L'additif selon l'invention peut en outre comprendre des ingrédients additionnels tels que des supports physiologiquement acceptables, des stabilisants, antioxydants, ou conservateurs, ainsi que des enzymes complémentaires telles que des protéases, des phytases, des mannanases, des amylases, des alpha-galactosidases, des acide férulique estérases et/ou des pectinases.

L'additif selon l'invention peut être sous toute forme adaptée pour son usage ultérieur, en particulier sous forme liquide, poudre ou granulé.

L'additif selon l'invention peut être utilisé comme ingrédient, en particulier comme ingrédient non-médicamenteux, de compositions alimentaires ou de prémix pour l'alimentation des ruminants tels que définis à la section *"Ruminants"* ci-dessus.

### Prémix

Par "prémix", on entend ici un concentré d'enzymes et éventuellement d'oligo-éléments, de vitamines et de minéraux, associés en faible pourcentage aux différentes matières premières pour constituer l'aliment complet à destination des ruminants.

Le prémix est de préférence constitué à partir d'une dilution ou mise sur support de l'additif tel que défini à la section *"Additif"* ci-dessus, pour standardiser l'activité enzymatique et faciliter son utilisation chez les animaux cibles.

La présente invention concerne ainsi un prémix pour l'alimentation des ruminants tels que définis à la section *"Ruminants"* ci-dessus, comprenant un additif tel que défini à la section *"Additif"* ci-dessus.

Le prémix selon l'invention peut comprendre d'autres additifs conventionnellement utilisés dans l'alimentation des animaux, en particulier des ruminants.

De tels additifs sont bien connus de l'homme du métier et incluent les additifs technologiques tels que les conservateurs, les antioxydants, les émulsifiants, les stabilisants, les épaississants, les gélifiants, les liants, les substances pour le contrôle de contamination de radionucléides, les anti-agglomérants, les correcteurs d'acidité, les additifs pour l'ensilage, les dénaturants; les additifs sensoriels tels que les colorants (dopant ou modifiant la couleur d'aliments pour animaux, des substances qui, incluses dans l'alimentation des animaux donneront une couleur aux denrées alimentaires produites à partir de ces animaux), des substances aromatiques; les additifs nutritionnels, tels que les vitamines, provitamines, acides gras oméga 3, et *"substances à effet analogue chimiquement bien définies",* les composés d'oligo-éléments, les acides aminés, l'urée; les acides gras et les additifs zootechniques tels que les améliorateurs de digestibilité comme des enzymes, des stabilisateurs de la flore intestinale, ou des substances ayant un *"effet positif sur l'environnement".*

A titre d'exemple, le prémix selon l'invention peut présenter la composition suivante :

| | |
|---|---|
| Composition enzymatique selon l'invention | 0,3 - 3% |
| Mélange de vitamines (A, B, D, E, acide nicotinique, etc) | 0,1 - 1% |
| Sels minéraux (CaHPO₄, CaCO₃, NaCl, CuO, MnO, FeSO₄, ZnO, etc) | 0 - 99,6% |
| Issues de céréales (son, remoulage, farine fourragère, rafle de maïs, radicelles d'orge, etc) | 0 - 99,6% |

Le prémix selon l'invention peut être sous toute forme adaptée pour son usage ultérieur, en particulier sous forme liquide, poudre ou granulé.

### Composition alimentaire

La présente invention concerne également une composition alimentaire supplémentée pour ruminants tels que définis à la section *"Ruminants"* ci-dessus, comprenant une quantité efficace d'un additif tel que défini à la section *"Additif"* ci-dessus ou d'un prémix tel que défini à la section *"Prémix"* ci-dessus, en association avec des aliments appropriés pour les ruminants tels que définis à la section *"Ruminants"* ci-dessus.

Les aliments appropriés pour les ruminants sont bien connus de l'homme du métier et incluent par exemple les fourrages de tous types et sous toutes leurs formes (verts, déshydratés, ensilés, agglomérés, etc) comme l'herbe et les autres graminées fourragères, les céréales fourragères (orge, maïs, avoine, blé, sorgho, soja, seigle), les légumineuses (pois, féverole, lupin, soja, luzerne, sainfoin, trèfles), les racines, tubercules et leurs sous-produits (betteraves, pulpe de betteraves, pomme de terre, pulpe de pomme de terre, etc), le chou, le colza, le tournesol, les déchets de végétaux (fanes, rafles, balles de céréales, son, épis de maïs égrenés, bagasse) et les fécules, les sous-produits d'industrie agro-alimentaire (amidonnerie, féculerie, éthanolerie, brasserie, meunerie, etc), ainsi que des tourteaux de graines oléagineuses, des sirops, et des matières alimentaires azotées telles que l'urée et ses dérivés (biuret, uréides) et les sels ammoniacaux.

De préférence, les aliments appropriés pour les ruminants utilisés dans le cadre de l'invention comprennent des fourrages, de préférence de l'ensilage de maïs représentant typiquement de 10 à 50% de la matière sèche ingérée, de préférence quotidiennement, associé de préférence à d'autres fourrages tels que du foin, de la paille ou de l'ensilage d'herbe ou de céréales et complémenté de préférence par des aliments concentrés tels que des céréales, des tourteaux d'oléagineux ou des aliments composés.

Une ration alimentaire pour ruminants peut notamment contenir la composition enzymatique telle que définie à la section *"Composition enzymatique"* ci-dessus avec :
- des fourrages humides, séchés ou secs en proportions allant de 0 à 100 %, de préférence de 40 à 70%.
- des aliments concentrés (matière première concentrée ou aliment composé) en proportions allant de 0 à 100%, de préférence de 30 à 60%.

De préférence, la composition enzymatique telle que définie à la section *"Composition enzymatique"* ci-dessus, l'additif tel que défini à la section *"Additif"* ci-dessus ou le prémix tel que défini à la section *"Prémix"* ci-dessus est présent dans la composition alimentaire à une quantité telle que la dose journalière efficace, telle que définie à la section *"Dose journalière efficace"* ci-dessous, est apportée à l'animal.

La composition alimentaire selon l'invention peut être sous toute forme adaptée pour l'alimentation des ruminants, en particulier des ruminants d'élevage. Elle peut en particulier être sous forme de flocon, granulé, de miette ou de farine.

La présente invention concerne également un procédé de fabrication d'une composition alimentaire supplémentée pour ruminants telle que définie ci-dessus comprenant une étape de mélange d'un additif tel que défini à la section *"Additif'* ci-dessus ou d'un prémix tel que défini à la section *"Prémix"* ci-dessus avec des aliments appropriés pour les ruminants, en particulier les ruminants d'élevage, tels que définis ci-dessus.

L'étape de mélange de l'additif ou du prémix avec l'aliment peut être mise en œuvre par toute technique bien connue de l'homme du métier.

Le procédé de fabrication de la composition alimentaire selon l'invention peut en outre comprendre une étape de formulation de la composition alimentaire ainsi qu'une étape de conditionnement. Ces étapes peuvent être mises en œuvre par toute technique conventionnelle bien connue de l'homme du métier. Ainsi, l'additif ou le prémix, quand ils sont sous forme de poudre, peuvent être ajoutés à l'aliment au moment de l'étape de formulation (ou mélange), ou lorsqu'ils sont sous forme de liquide, peuvent être pulvérisés après granulation de l'aliment.

### Dose journalière efficace

L'additif tel que défini à la section *"Additif"* ci-dessus, le prémix tel que défini à la section *"Prémix"* ci-dessus, ou la composition alimentaire telle que définie à la section *"Composition alimentaire"* ci-dessus, sont formulés de manière à apporter la quantité d'activités enzymatiques efficace chez les animaux cibles. La formulation de l'additif, du prémix, ou de la composition alimentaire peut être réalisée par toute technique bien connue de l'homme du métier, telles que notamment mélange, dilution, mise sur support, sprayage ou pulvérisation sur granulés, ou distribution directe aux animaux.

La formulation adoptée dépend de l'animal cible et est de préférence pilotée par le niveau d'activité β-glucanase.

Les doses journalières efficaces sont typiquement :
- vaches laitières : de 2250 à 9000 BGU / animal / jour, de préférence 4500 BGU / animal / jour
- bovins à l'engrais : de 2250 à 9000 BGU / animal / jour, de préférence 2250 BGU / animal / jour jusqu'à 500 kg de poids vif et 3375 BGU / animal / jour au-delà de 500 kg de poids vif.

Les doses journalières efficaces pour les autres types de ruminants peuvent être calculées par l'homme du métier par comparaison avec les vaches laitières ou les bovins à l'engrais, selon des techniques bien connues de l'homme du métier, telles que notamment en fonction du poids vif, en fonction du poids vif métabolique (PV^{0.75}), en fonction du volume du rumen, en fonction de la quantité totale de matière sèche ingérée par jour, avec une quantité minimale de préférence de 1125 BGU / animal / jour, particulièrement chez les animaux dont le poids vif est inférieur à 100 kg.

### Utilisations pour augmenter les performances zootechniques

Les inventeurs ont montré que supplémenter la ration alimentaire de vaches laitières ou de bovins à l'engrais avec la composition enzymatique selon l'invention permettait de manière surprenante d'accroître leurs performances zootechniques, en particulier d'améliorer la production laitière, y compris le taux butyreux et le taux protéique du lait produit, d'augmenter le gain de poids, d'augmenter l'activité de rumination, d'améliorer l'efficacité alimentaire et l'indice de consommation, d'augmenter la capacité d'ingestion des fourrages, d'augmenter la digestibilité des fourrages et/ou des concentrés et de réduire la production de méthane.

La présente invention a donc également pour objet l'utilisation non-thérapeutique d'une composition enzymatique telle que définie à la section *"Composition enzymatique"* ci-dessus, d'un additif tel que défini à la section *"Additif"* ci-dessus, d'un prémix tel que défini à la section *"Prémix"* ci-dessus ou d'une composition alimentaire telle que définie à la section *"Composition alimentaire"* ci-dessus, pour augmenter les performances zootechniques d'un ruminant tel que défini à la section *"Ruminants"* ci-dessus.

Par "performance zootechnique", on entend ici un indicateur permettant de juger de la qualité d'un animal, en particulier de son aptitude biologique pour différentes fonctions (croissance, travail, reproduction...). Les performances zootechniques incluent en particulier le gain de poids de l'animal, en particulier le gain de poids moyen quotidien, son indice de consommation ou son efficacité alimentaire, le poids à âge-type, le rendement à l'abattage, le poids de carcasse, la conformation de carcasse, la prise alimentaire, la capacité d'ingestion des fourrages, la digestibilité des fourrages et/ou des concentrés, la production de lait, le taux protéique du lait, le taux de matières grasses du lait, l'activité de rumination, la production de méthane, la taille de portée ou la production de poils. De préférence, la composition enzymatique telle que définie à la section *"Composition enzymatique"* ci-dessus, l'additif tel que défini à la section *"Additif"* ci-dessus, le prémix tel que défini à la section *"Prémix"* ci-dessus ou la composition alimentaire telle que définie à la section *"Composition alimentaire"* ci-dessus permet de favoriser le gain de poids et/ou d'améliorer l'indice de consommation et/ou favoriser la prise alimentaire et/ou d'améliorer le rendement à l'abattage, le poids de carcasse, la conformation de carcasse, la production de lait, le taux protéique du lait, le taux de matières grasses du lait, la taille de portée et/ou la production de poils et/ou d'augmenter l'activité de rumination et/ou d'augmenter la capacité d'ingestion des fourrages et/ou d'augmenter la digestibilité des fourrages et/ou des concentrés et/ou de réduire la production de méthane chez un ruminant tel que défini à la section *"Ruminants"* ci-dessus. De manière particulièrement préférée, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet d'augmenter la production laitière et/ou d'améliorer l'indice de consommation et/ou de favoriser le gain de poids et/ou d'augmenter l'activité de rumination et/ou d'augmenter la capacité d'ingestion des fourrages et/ou d'augmenter la digestibilité des fourrages et/ou des concentrés et/ou de réduire la production de méthane chez un ruminant tel que défini à la section *"Ruminants"* ci-dessus. De manière préférée entre toutes, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet d'augmenter la production laitière et/ou d'améliorer l'indice de consommation et/ou de favoriser le gain de poids et/ou d'augmenter l'activité de rumination chez un ruminant tel que défini à la section *"Ruminants"* ci-dessus

Par "gain de poids" ou "gain moyen quotidien", on entend ici l'évolution de la croissance pondérale de l'animal, exprimée en g/jour.

Par "indice de consommation", on entend ici l'efficacité de la conversion alimentaire. Cet indice est déterminé par le rapport entre la quantité d'aliments consommés et le gain de poids vif dans le cas des ruminants à l'engrais, et par le rapport entre la quantité d'aliments consommés et la production laitière dans le cas des ruminants laitiers.

Par "efficacité alimentaire", on entend ici la mesure de la transformation de la ration alimentaire des ruminants en produits. Il s'agit du rapport inverse de l'indice de consommation défini ci-dessus.

Par "poids à âge-type", on entend ici le poids de l'animal à un âge de référence (1 an ou 18 mois par exemple), ce qui permet de faciliter les comparaisons.

Par "rendement à l'abattage", on entend ici le rapport entre le poids de la carcasse et le poids vif de l'animal, ce qui permet d'estimer le "rendement vrai".

Par "poids de carcasse", on entend ici le poids de la carcasse après ressuyage.

Par "prise alimentaire", on entend la quantité d'aliments ingérés par l'animal.

Par "capacité d'ingestion des fourrages", on entend la quantité de fourrages spontanément ingérée par l'animal.

Par "digestibilité des fourrages et/ou des concentrés", on entend la part de fourrages et/ou concentrés effectivement retenue ou digérée par l'animal.

Par "production de lait" ou "production laitière", on entend la quantité de lait produite par jour, exprimée en unité de volume ou en unité de poids. Dans le contexte de l'invention, l'amélioration de la production laitière peut se traduire par une augmentation de la quantité de lait produite, mais également par une amélioration du taux butyreux et/ou du taux protéique du lait.

Par "taux protéique du lait", on entend ici la quantité de matière protéique contenue dans le lait.

Par "taux de matières grasses du lait", on entend ici la quantité de matières grasses contenue dans le lait.

Par "taille de portée", on entend l'effectif et le poids à la naissance des jeunes ou des petits.

Par "activité de rumination", on entend ici le nombre de minutes par jour consacrées à la mastication du bol alimentaire.

Par "production de poils", on entend la quantité de poils produite par un mammifère élevé pour ses poils (chèvre cachemire par exemple).

De préférence, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet d'améliorer la production laitière, en particulier une amélioration du taux butyreux et/ou du taux protéique du lait, et/ou d'améliorer l'indice de consommation et/ou l'efficacité alimentaire et/ou de favoriser le gain de poids et/ou d'augmenter l'activité de rumination et/ou d'augmenter la capacité d'ingestion des fourrages et/ou d'augmenter la digestibilité des fourrages et/ou des concentrés et/ou de réduire la production de méthane de manière significative chez un ruminant tel que défini à la section *"Ruminants"* ci-dessus.

En particulier, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet d'améliorer la production laitière de 1 à 5%, de préférence de 3%, et/ou d'améliorer le taux protéique du lait de 0,5 à 1,5%, de préférence de 1%, chez un ruminant, en particulier chez un bovin, de préférence chez une vache laitière, nourri de préférence avec un aliment comprenant de l'ensilage de maïs, de préférence un aliment comprenant un fourrage grossier tel que la paille ou le foin, des tourteaux de graines oléagineuses, de la pulpe de betterave et des concentrés azoté et énergétique, la composition enzymatique telle que définie à la section *"Composition enzymatique"* ci-dessus étant de préférence présente dans l'aliment de manière à apporter 4500 BGU/animal/jour.

En particulier, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet également d'améliorer le gain de poids de 1 à 15%, de préférence de 10%, chez un ruminant, en particulier chez un bovin, de préférence chez un bovin à l'engrais, nourri de préférence avec un aliment comprenant de l'ensilage de maïs, de préférence un aliment comprenant de l'ensilage de maïs et des concentrés azoté et énergétique, la composition enzymatique telle que définie à la section *"Composition enzymatique"* ci-dessus étant de préférence présente dans l'aliment de manière à apporter 2250 BGU / animal / jour jusqu'à 500 kg de poids vif et 3375 BGU / animal / jour au-delà de 500 kg de poids vif.

En particulier, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet également d'augmenter l'activité de rumination de 5 à 15%, de préférence de 8 à 11%, chez un ruminant, en particulier chez un bovin, de préférence chez une vache laitière, nourri de préférence avec un aliment comprenant de l'ensilage de maïs, de préférence un aliment comprenant de l'ensilage de maïs et des concentrés azoté et énergétique, la composition enzymatique telle que définie à la section *"Composition enzymatique"* ci-dessus étant de préférence présente dans l'aliment de manière à apporter 4500 BGU/animal/jour.

En particulier, la composition enzymatique telle que définie à la section *"Composition enzymatique"* ci-dessus, l'additif tel que défini dans la section *"Additif"* ci-dessus, le prémix tel que défini à la section *"Prémix"* ci-dessus ou la composition alimentaire telle que définie à la section *"Composition alimentaire"* ci-dessus permet également de réduire la production de méthane de 0,5 à 9%, de préférence de 2,5 à 8%, chez un ruminant, en particulier chez un bovin, de préférence chez une vache réformée, nourri de préférence avec un aliment comprenant du foin d'herbe, de l'ensilage de maïs et/ou de l'ensilage d'herbe, de préférence un aliment comprenant du foin d'herbe, la composition enzymatique telle que définie à la section *"Composition enzymatique"* ci-dessus étant de préférence présente dans l'aliment de manière à apporter 3375 BGU/animal/jour.

La composition enzymatique telle que définie à la section *"Composition enzymatique"* ci-dessus, l'additif tel que défini à la section *"Additif"* ci-dessus, le prémix tel que défini à la section *"Prémix"* ci-dessus et la composition alimentaire telle que définie à la section *"Composition alimentaire"* ci-dessus, permettent en outre de maintenir un bon état général du ruminant ou du troupeau de ruminants au(x)quel(s) ils sont administrés, et en particulier de maintenir une fertilité, une morbidité et/ou une mortalité du troupeau acceptable pour l'exploitant, voire de favoriser cette fertilité et/ou de réduire cette morbidité et/ou cette mortalité.

La présente invention concerne également un procédé non-thérapeutique pour augmenter les performances zootechniques d'un ruminant caractérisé en ce que l'on fait ingérer au ruminant, de préférence dans la ration alimentaire, une quantité efficace de la composition enzymatique telle que définie à la section *"Composition enzymatique"* ci-dessus, de l'additif tel que défini à la section *"Additif"* ci-dessus, du prémix tel que défini à la section *"Prémix"* ci-dessus ou de la composition alimentaire telle que définie à la section *"Composition alimentaire"* ci-dessus.

Par "quantité efficace", on entend ici la quantité de composition enzymatique, éventuellement présente dans l'additif, le prémix ou la composition alimentaire, permettant d'accroître les performances zootechniques telles que définies ci-dessus. Comme il sera clairement apparent pour l'homme du métier, cette quantité dépendra du ruminant considéré, de son âge, de son sexe, de son type génétique, de son stade physiologique, de son poids, de son niveau de performance attendue et de sa ration alimentaire.

Typiquement, dans le cas des bovins, la quantité ingérée efficace est de telle sorte que l'activité β-glucanase ingérée est comprise entre 2250 et 9000 BGU/jour, de préférence 4500 BGU/jour pour une vache laitière, et entre 2250 à 9000 BGU/jour pour un bovin à l'engrais, de préférence 2250 BGU/jour pour un bovin à l'engrais ayant jusqu'à 500 kg de poids vif et 3375 BGU/jour pour un bovin à l'engrais ayant plus de 500 kg de poids vif.

De manière générale, la composition enzymatique telle que définie à la section *"Composition enzymatique"* ci-dessus est fournie au ruminant sous la forme de prises successives et étalées dans le temps, par exemple selon des rythmes quotidiens, bihebdomadaires, hebdomadaires ou bien bi-mensuels. De manière préférée, on fait ingérer aux ruminants une quantité efficace de composition enzymatique telle que définie à la section *"Composition enzymatique"* ci-dessus selon une dose quotidienne.

Pour les ruminants qui sont nourris exclusivement au pâturage, la composition enzymatique telle que définie à la section *"Composition enzymatique"* ci-dessus sera de préférence fournie séparément de leur alimentation principale.

Pour les ruminants qui sont nourris avec du fourrage frais ou du fourrage conservé (*e.g.* du foin), ou bien avec des aliments industriels, y compris avec des concentrés alimentaires, la composition enzymatique telle que définie à la section *"Composition enzymatique"* ci-dessus peut être fournie en mélange avec ces aliments ou sous une forme séparée de ces aliments.

La présente invention sera illustrée plus en détail par les exemples ci-dessous.

### EXEMPLES

### Exemple 1 : Production de la composition enzymatique selon l'invention par fermentation en milieu solide

Dans cet exemple est décrite une méthode d'obtention de la composition enzymatique selon l'invention par fermentation en milieu solide d'un mélange tourteau de colza et son de blé en présence d'une souche d'*Aspergillus tubingensis.*

Un milieu nutritif est constitué par un mélange de tourteau de colza en farine et de son de blé dans une proportion 7/3 en poids. Le mélange est ensuite pré-humidifié à 60% de matière sèche et autoclavé pendant 35 min à 105°C. Après refroidissement, le milieu est inoculé par une solution de spores d'*Aspergillus tubingensis* afin d'obtenir une concentration de 1×10⁷ spores par gramme de matière sèche et une humidité initiale de 45%. Le pH est ajusté à 4,9 par ajout d'acide sulfurique. Le milieu de culture ainsi obtenu est réparti dans des fioles d'erlenmeyer à raison de 10 g de matière sèche par fiole. Les fioles d'erlenmeyer sont ensuite incubées à 33°C en conditions aérobies à l'obscurité pendant 48 h sans agitation. La culture est arrêtée à l'apparition des premières spores dans le milieu de culture (la présence de spores pouvant gêner les animaux lors de l'ingestion du fait de leur caractère volatil, les produits fermentés sont conçus de manière à en contenir le moins possible).

Le mélange obtenu en fin de fermentation peut être séché et utilisé tel quel dans l'alimentation des animaux ou être extrait dans l'eau pour être ajouté sous forme liquide.

### Exemple 2 : Mesure de l'effet de la composition enzymatique selon l'invention sur les performances de production de vaches laitières

Dans cet exemple est décrit l'effet de la composition enzymatique selon l'invention sur les performances de production d'un troupeau de vaches laitières hautes productrices.

### Matériels et méthodes

38 vaches laitières hautes productrices de race Prim'Holstein ont été réparties dans 2 groupes et appariées. La mise en lot a été effectuée sur les critères suivants : production laitière, taux protéique du lait, taux de matières grasses du lait, rang de lactation, poids vif.

Les animaux ont reçu une ration mélangée composée de 9,5 kg MS (Matière sèche) d'ensilage de maïs, 2,1 kg MS d'aliment fibreux (paille + luzerne brins longs), 4,9 kg MS de correcteur azoté (dont la composition est décrite dans le tableau 1), 8,0 kg d'aliment de production (dont la composition est décrite dans le tableau 1).

La composition enzymatique, obtenue par le procédé décrit dans l'exemple 1, a été standardisée sous forme d'un prémix pour assurer une bonne homogénéité de la composition et faciliter son utilisation lors de l'étape de fabrication des aliments. Le prémix ainsi standardisé a été incorporé dans l'aliment de production à raison de 0,25% pour apporter 4500 BGU/animal/jour. Les caractéristiques du prémix ainsi standardisé sont : 130 AXC/g, 290 BGU/g et 35 CMC/g. La durée de l'essai est de 8 semaines.

Un apport de 1 kg/jour d'un aliment contenant du monopropylèneglycol (Sandi+, une source énergétique) est réalisé sur les vaches de moins de 30 jours de lactation.

Les caractéristiques moyennes de la ration sont (en %/sec) : Matières Azotées totales : 17,7%; Fibres insolubles dans les détergents neutres (NDF) : 35,8% et Amidon : 17,6%.

**Tableau 1 : Composition des aliments pour vaches laitières**

| **Matières premières (%)** | **Correcteur azoté** | **Aliment de production (contrôle)** | **Aliment de production (+ composition enzymatique selon l'invention)** | **Sandi+** |
|---|---|---|---|---|
| Tourteau colza Expeller | 18,0 | | | |
| Orge | 18,5 | 15,2 | 15,3 | 42,3 |
| Tourteau soja | 52,9 | 5,8 | 6,6 | |
| Graine colza | | 7,8 | 7,9 | |
| Tourteau colza | | 24,8 | 24,3 | 32,6 |
| Coque soja | | 20,0 | 20,0 | |
| Pulpe betterave | | 22,0 | 21,6 | |
| Paille | | 2,0 | 2,0 | |
| Son | | | | 15,1 |
| Huile de soja | 1,7 | | | |
| Bicarbonate Na | 1,3 | 1,5 | 1,5 | |
| Complément minéralisé ou vitaminé (CMV) | 3,9 | 0,3 | 0,3 | |
| Urée | 1,1 | | | |
| Prémix | 0,5 | 0,5 | 0,5 | |
| Monopropylèneglyc ol | | | | 10,0 |
| Blé | 2,3 | 0,3 | | |
| ***Composition enzymatique selon l'invention*** | | | 0,25 | |
| Matière sèche (%) | 90,1 | 88,7 | 88,7 | 88,2 |
| Protéine (% brut) | 35,0 | 18,0 | 18,0 | 18,0 |
| Matières grasses (% brut) | 5,0 | 5,1 | 5,1 | 2,2 |
| Cellulose brute (%) | 6,4 | 16,4 | 16,4 | 8,0 |
| NDF (% brut) | 16,4 | 35,0 | 35,0 | 24,0 |
| Fibres insolubles dans les détergents acides (ADF) (% brut) | 9,2 | 21,1 | 21,0 | 11,0 |
| Lignine (ADL) (% brut) | 2,0 | 3,4 | 3,4 | 3,5 |
| UF lait (/kg brut) | 100,1 | 95,0 | 95,0 | 90,0 |
| PDIA (g/kg brut) | 116,1 | 55,7 | 56,0 | 48,6 |
| PDIN (g/kg brut) | 245,2 | 119,7 | 119,8 | 117,9 |
| PDIE (g/kg brut) | 164,3 | 106,5 | 106,9 | 97,5 |

Les caractéristiques des lots d'animaux sont décrites dans le tableau 2.

**Tableau 2 : Caractéristiques des lots de vaches laitières à la mise en lot (après 2 semaines pré-expérimentales)**

| | Témoin | Traitement |
|---|---|---|
| Nombre animaux | 19 | 19 |
| Stade de lactation (j) | 168 | 185 |
| Production Laitière (kg/j) | 39.5 | 39.5 |
| Taux Butyreux (g/kg) | 35.9 | 35.9 |
| Taux Protéique (g/kg) | 31.6 | 32.1 |
| Cellule Somatique du lait (1000/mL) | 391 | 220 |
| Urée (mg/L) | 267 | 284 |
| Poids Vif (kg) | 658 | 670 |

### Résultats

Les résultats de production des deux groupes sont détaillés dans le tableau 3.

**Tableau 3 : Résultats de production des lots et statistiques**

| | Témoin | Traitement | Effet Lot |
|---|---|---|---|
| Nombre animaux | 19 | 19 | |
| Production Laitière (PL) (kg/j) | 39.3 | 40.4 | *** |
| Taux Butyreux (TB) (g/kg) | 35.8 | 35.6 | NS |
| Taux Protéique (TP) (g/kg) | 31.0 | 31.3 | ** |
| Matière Grasse Exportée (g/j) | 1395 | 1413 | NS |
| Matière Protéique Exportée (g/j) | 1210 | 1244 | *** |
| Production Laitière corrigée 7% (kg/j) | 37.2 | 38.0 | ** |
| Cellule Somatique du lait (1000/mL) | 94 | 106 | NS |
| Poids Vif (kg) | 682 | 671 | *** |

| | | | |
|---|---|---|---|
| NS : non significatif ; * : p<0.10 ; ** : p<0.05 ; *** : p<0.001 | | | |

Les inventeurs ont constaté que l'apport de composition enzymatique a un effet très marqué sur de nombreux critères :
- hausse significative de la production laitière ;
- hausses significatives du taux protéique (+0,3 g/kg), et de la matière protéique produite ;
- hausse significative de la PL7% (production laitière corrigée des taux = PL * (TB + TP)/70) : +0,7 kg.

Ces effets ne sont pas accompagnés d'une augmentation de la matière sèche ingérée et traduisent donc une meilleure efficacité alimentaire (quantité de lait produit / matière sèche ingérée).

Cet exemple démontre que l'apport de composition enzymatique selon l'invention permet une amélioration des performances zootechniques de production de lait et une meilleure valorisation de la ration alimentaire des vaches laitières hautes productrices.

### Exemple 3 : Mesure de l'effet de la composition enzymatique selon l'invention sur les performances de croissance de bovins à l'engrais

Dans cet exemple est décrit l'effet de la composition enzymatique selon l'invention sur les performances de croissance de bovins à l'engrais.

### Matériels et méthodes

40 bovins à l'engrais de race Charolais ont été répartis dans deux groupes. La mise en lot a été effectuée sur le critère du poids vif.

Les animaux ont reçu une ration complète mélangée dont la composition est détaillée dans le Tableau 4.

La composition enzymatique, obtenue par le procédé décrit dans l'exemple 1, a été standardisée sous forme d'un prémix pour assurer une bonne homogénéité de la composition et faciliter son utilisation lors de l'étape de distribution aux animaux. Le prémix ainsi standardisé a été incorporé dans la ration complète mélangée pour apporter 9000 BGU/animal/jour. Les caractéristiques du prémix ainsi standardisé sont : 80 AXC/g, 150 BGU/g et 17 CMC/g.

L'essai a duré 10 mois.

**Tableau 4 : Composition de la ration complète mélangée pour bovins à l'engrais**

| | Quantité distribuée (kg brut/animal/jour) | |
|---|---|---|
| Ingrédient | Engraissement de 300 à 500 kg de poids vif | Finition de 500 à 750 kg de poids vif |
| Pulpe de pomme de terre | 3,0 | 3,0 |
| Paille de blé | 0,8 | 0,8 |
| Enrubanné de luzerne (33% Matière Sèche) | 3,0 | 3,0 |
| Concentré azoté (44% MAT) | 0,8 | 0,65 |
| Concentré énergétique (1 UFV/kg) | 2,5 | 3,5 |
| Minéraux | 0,1 | 0,1 |
| Urée | 0,01 | 0,06 |
| Maïs ensilage et pulpes de | 14,0 | 20,0 |
| betteraves surpressées (50/50 en matière sèche) | | |

### Résultats

Les résultats de production des deux groupes sont détaillés dans le tableau 5.

**Tableau 5 : Résultats de croissance des lots**

| GMQ (g/animal/jour) | | Naissance à T0 (8.3 mois en moyenne) | T0 à T0+1 mois | T0+1 mois à T0+2mois | T0+2mois à T0+8mois | T0 à T0+8mois | Poids Carcasse (kg) |
|---|---|---|---|---|---|---|---|
| Témoin | Moyenne | 1 139 | 1 564 | 1 633 | 1 095 | 1 219 | 415 |
| | Ecart-type | 139 | 369 | 340 | 236 | 186 | 30 |
| Traitement | Moyenne | 1 139 | 1 708 | 1 709 | 1 218 | 1 342 | 425 |
| | Ecart-type | 117 | 590 | 293 | 197 | 200 | 39 |
| Amélioration induite par le traitement en base 100 du témoin (% d'amélioration) | | 100 (0%) | 109 (9%) | 105 (5%) | 111 (11%) | 110 (10%) | 102 (2%) |

Les inventeurs ont constaté que l'ajout de la composition enzymatique dans l'alimentation de jeunes bovins à l'engrais a permis une amélioration de 10% du Gain de poids Moyen Quotidien (GMQ). Ceci s'est traduit par un poids de carcasse à l'abattage amélioré de 2% dans le lot d'animaux supplémenté avec la composition enzymatique selon l'invention.

Cet exemple démontre que l'apport de la composition enzymatique selon l'invention permet une amélioration des performances zootechniques de croissance de bovins à l'engrais.

### Exemple 4 : Mesure de l'effet de la composition enzymatique selon l'invention sur les performances de production et l'activité de rumination de vaches laitières

Dans cet exemple est décrit l'effet de la composition enzymatique selon l'invention sur les performances de production d'un troupeau de vaches laitières hautes productrices et sur l'activité de rumination des animaux.

### Matériels et méthodes

36 vaches laitières hautes productrices de race Prim'Holstein ont été réparties dans deux groupes. La mise en lot des animaux a été effectuée sur les critères de production laitière, poids vif, rang de lactation et jours en lactation. L'essai a duré 85 jours.

Les animaux ont reçu une ration complète mélangée, principalement composée d'ensilage de maïs, foin de luzerne, foin de ray-gras, « corn gluten feed », concentré de production et d'eau. La composition détaillée est donnée dans le tableau 6.

La composition enzymatique, préparée selon le procédé de production décrit dans l'exemple 1, a été standardisée sous forme d'un prémix pour assurer une bonne homogénéité de la composition et faciliter son utilisation lors de l'étape de distribution aux animaux (distribution en l'état, en « top feeding »). Le prémix obtenu a été mélangé dans la ration totale de telle sorte à apporter 4500 BGU/animal/jour. Les caractéristiques du prémix ainsi standardisé sont : 400 AXC/g, 270 BGU/g et 69 CMC/g.

L'activité de rumination des 36 vaches a été enregistrée quotidiennement par un détecteur acoustique (RuminAct, Milkline, Italie).

**Tableau 6 : Composition de la ration complète mélangée des vaches laitières**

| | | |
|---|---|---|
| Ensilage de maïs | kg | 22,0 |
| Concentré¹ + tourteau de coton | kg | 6,0 |
| Corn gluten meal-orge floconné mix. (60:40) | kg | 6,8 |
| Eau | kg | 8,0 |
| Foin de ray-gras | kg | 1,1 |
| Foin de luzerne | kg | 4,4 |

| | | |
|---|---|---|
| ¹: tourteau de soja 44% : 41,2%; tourteau de coton : 33%, tourteau de tournesol : 7,21%; Corn gluten meal :7,21% ; CaCO₃ : 2,68% ; NaHCO₃ : 2,68% ; MgO : 1,00% ; CaHPO₄ : 0,67% ; NaCl : 0,67% ; ZnSO₄: 0,10% ; Premix : 0,54%. | | |

Les valeurs nutritionnelles de la ration complète mélangée ont été analysées : matière sèche 48,69%, Protéines brutes : 15,75%/ matière sèche (sec) ; Matières grasses: 2,70%/sec ; Cellulose brute: 18,45%/sec; NDF : 37,86%/sec ; ADF : 21,15%/sec et amidon : 25,57 %/sec.

Il n'y a pas eu de différence d'ingestion entre les deux lots au cours de cet essai.

### Résultats

Les résultats de production laitière des deux groupes sont détaillés dans le tableau 7.

**Tableau 7 : Résultats de production laitière (kg/animal/jour) des lots et statistiques**

| Périodes | Contrôle | Traitement | Traitement *vs*. Contrôle | Effet du traitement P= | Effet de la Période P= | Interaction (Traitement x Période) P= |
|---|---|---|---|---|---|---|
| J0 - J21 | 36,7±8,5 | 37,5±8,4 | +0,8 | 0,7840 | **0,0001** | 0,9896 |
| J21 - J37 | 36,9±8,4 | 38,3±8,6 | +1,4 | 0,6135 | **0,0001** | 0,4114 |
| J37 - J53 | 35,6±9,2 | 37,6±8,1 | +2,0 | 0,4786 | **0,0001** | 0,1807 |
| J53 - J69 | 34,1±8,7 | 35,5±8,1 | +1,4 | 0,6024 | 0,1024 | 0,9907 |
| J69 - J85 | 33,7±9,2 | 35,0±8,0 | +1,3 | 0,6424 | **0,0001** | 0,9938 |
| J21 - J85 | 35,1±8,9 | 36,6±8,3 | +1,5 | 0,5795 | **0,0001** | 0,9472 |

La production laitière a diminué au cours de l'essai en rapport avec le stade de lactation. Durant l'essai, le lot recevant la composition enzymatique selon l'invention a montré une amélioration de production laitière de +1,5 kg de lait par jour et par animal par rapport au groupe Contrôle.

Les résultats de composition du lait (matières grasse et protéique) sont détaillés dans le tableau 8.

**Tableau 8 : Composition du lait des lots et statistiques**

| Jour | Contrôle | Traitement | Traitement *vs*. Contrôle | Effet Traitement P= | Erreur Standard |
|---|---|---|---|---|---|
| Taux butyreux du lait (%) | | | | | |
| J0 | 3,99±0,52 | 3,87±0,54 | -0,12 | 0,5102 | 0,1251 |
| J21 | 4,01±0,74 | 4,00±0,58 | -0,01 | 0,9421 | 0,1558 |
| J37 | 3,98±0,49 | 4,02±0,49 | +0,04 | 0,7988 | 0,1162 |
| J53 | 4,12±0,73 | 4,34±0,61 | +0,22 | 0,3368 | 0,1581 |
| J69 | 4,15±0,54 | 4,30±0,69 | +0,15 | 0,4860 | 0,1462 |
| J85 | 4,09±0,62 | 4,21±0,55 | +0,12 | 0,5305 | 0,1376 |

| Production de matières grasses laitières (kg/jour) | | | | | |
|---|---|---|---|---|---|
| J0 | 1,42±0,33 | 1,38±0,29 | -0,04 | 0,7318 | 0,0731 |
| J21 | 1,42±0,25 | 1,47±0,31 | +0,05 | 0,5751 | 0,0662 |
| J37 | 1,44±0,23 | 1,51±0,27 | +0,07 | 0,4430 | 0,0585 |
| J53 | 1,38±0,29 | 1,51±0,33 | +0,13 | 0,2094 | 0,0729 |
| J69 | 1,37±0,31 | 1,48±0,26 | +0,11 | 0,2739 | 0,0675 |
| J85 | 1,26±0,23 | 1,34±0,22 | +0,08 | 0,2790 | 0,0535 |

| Taux protéique du lait (%) | | | | | |
|---|---|---|---|---|---|
| J0 | 3,57±0,40 | 3,46±0,31 | -0,11 | 0,3732 | 0,0844 |
| J21 | 3,60±0,45 | 3,78±0,22 | +0,18 | 0,1243 | 0,0832 |
| J37 | 3,66±0,32 | 3,80±0,43 | +0,14 | 0,2724 | 0,0887 |
| J53 | 3,66±0,33 | 3,87±0,22 | +0,21 | 0,0324 | 0,0671 |
| J69 | 3,67±0,30 | 3,84±0,23 | +0,17 | 0,0659 | 0,0624 |
| J85 | 3,69±0,31 | 3,82±0,31 | +0,13 | 0,2092 | 0,0727 |

| Production de protéines laitières (kg/jour) | | | | | |
|---|---|---|---|---|---|
| J0 | 1,26±0,19 | 1,24±0,22 | -0,02 | 0,7599 | 0,0490 |
| J21 | 1,29±0,23 | 1,42±0,35 | +0,13 | 0,2103 | 0,0705 |
| J37 | 1,34±0,30 | 1,41±0,19 | +0,07 | 0,4245 | 0,0590 |
| J53 | 1,24±0,27 | 1,37±0,35 | +0,13 | 0,2293 | 0,0729 |
| J69 | 1,23±0,32 | 1,34±0,29 | +0,11 | 0,2692 | 0,0708 |
| J85 | 1,15±0,253 | 1,23±0,24 | +0,08 | 0,3567 | 0,0579 |

Pendant l'essai, le taux butyreux est resté stable dans le groupe Contrôle pendant que celui-ci augmentait dans le groupe Traitement (supplémenté avec la composition enzymatique selon l'invention). Le taux butyreux a toujours été plus élevé dans le lot de vaches traitées que dans le lot de vaches contrôle. Après 85 jours d'essai, les vaches du groupe Traitement présentent un taux butyreux supérieur de 0,12 point à celui des vaches du groupe Contrôle.

Au cours de l'essai, le taux de protéines du lait a augmenté plus vite dans le groupe Traitement que dans le groupe Contrôle. Statistiquement, les inventeurs ont constaté une hausse significative du taux protéique à J53 et à J69, +0,21 et +0,17 point respectivement. Ceci se traduit également par une exportation de protéines plus importante (+0,08 kg/jour à la fin de l'essai).

L'activité de rumination est d'une grande importance pour l'activité métabolique des vaches laitières et peut être un outil utile pour la surveillance de la santé animale. La rumination stimule la production de salive et assure donc des conditions optimales pour l'activité cellulolytique dans le rumen.

Les données d'activité de rumination quotidienne moyennes sont données dans le tableau 9. L'activité de rumination est exprimée en nombre de minutes de rumination par jour et par animal.

**Tableau 9 : Activité de rumination des animaux (minutes de rumination/animal/jour)**

| Périodes | Contrôle | Traitement | Traitement *vs*. Contrôle | Effet du traitement P= | Effet de la Période P= | Interaction (Traitement x Période) P= |
|---|---|---|---|---|---|---|
| J0 - J21 | 500,6 | 511,6 | +11,0 | 0,6513 | **0,0001** | 0,4360 |
| J21 - J37 | 497,0 | 515,8 | +18,8 | 0,4730 | **0,0001** | 0,6990 |
| J37 - J53 | 458,3 | 497,8 | +39,5 | 0,1858 | **0,0001** | 0,2840 |
| J53 - J69 | 482,3 | 528,1 | +45,9 | 0,1210 | **0,0322** | 0,3747 |
| J69 - J85 | 471,7 | 523,9 | +52,2 | 0,1019 | **0,0001** | **0,0132** |
| J21 - J85 | 477,3 | 516,4 | +39,1 | 0,1657 | **0,0001** | **0,0066** |

Les deux groupes ne présentent pas de différence statistique au début de l'essai (500,6 *vs.* 511,6 min/jour/animal pour les groupes Contrôle et Traitement respectivement). Néanmoins, après 16 jours d'essai, l'activité de rumination augmente numériquement dans le lot Traitement. Dans la dernière période de l'essai (J69-J85), l'interaction (traitement x période) est significative (P=0,0132) avec une activité de rumination du groupe Traitement supérieure à celle du groupe Contrôle (+52,2 minutes/jour/animal). L'analyse statistique sur la période J21-J85 montre le même effet d'interaction avec +39 min/jour/animal d'activité de rumination chez les vaches du groupe Traitement.

Cet exemple démontre que l'apport de composition enzymatique selon l'invention permet une amélioration des performances zootechniques de production de lait (production laitière quotidienne, production de protéines laitières et de matières grasses laitières), une meilleure valorisation de la ration alimentaire des vaches laitières hautes productrices et une activité de rumination améliorée.

### Exemple 5 : Mesure de l'effet de la composition enzymatique selon l'invention sur les fermentations ruminales in vitro (dégagements gazeux)

Dans cet exemple est décrit l'effet de la composition enzymatique selon l'invention sur les fermentations ruminales *in vitro,* en particulier les dégagements gazeux et notamment la production de méthane.

### Matériels et méthodes

### Animaux

Deux vaches réformées canulées au rumen ont été utilisées. Pendant l'entièreté de la durée de l'expérience *in vitro,* les animaux ont été nourris avec un mélange d'herbe préfanée ensilée (4 kg), d'ensilage de maïs (5 kg/j), paille hachée (1 kg) et de concentré (1kg).

### Ingrédients

Quatre aliments ont été étudiés *in vitro* : Foin d'herbe, Ensilage d'herbe, Ensilage de maïs et Tourteau de soja. La composition chimique des ingrédients expérimentaux est présentée dans le Tableau 10.

**Tableau 10 : Composition chimique des ingrédients utilisés**

| Ingrédients | MAT¹ (%) | NDF(%) | Amidon(%) | Energie brute (kcal/kg MS) |
|---|---|---|---|---|
| Soja | 55,37 | 15,18 | N/A² | 4636,92 |
| Préfané | 17,90 | 44,72 | N/A | 4106,45 |
| Maïs | 5,07 | 53,53 | 19,5 | 4546,62 |
| Foin | 6,89 | 65,93 | N/A | 4306,68 |

| | | | | |
|---|---|---|---|---|
| ¹MAT: Matière azote totale ²N/A : Non applicable | | | | |

Les différents ingrédients ont été lyophilisés et moulus à 1 mm au moyen d'un moulin de laboratoire.

La composition enzymatique, préparée selon le procédé de production décrit dans l'exemple 1 en utilisant une souche d'*Aspergillus neoniger,* a été standardisée sous forme d'un prémix pour assurer une bonne homogénéité de la composition et faciliter son utilisation lors de l'étape de distribution aux animaux en « top feeding ». Le prémix obtenu a été mélangé dans la ration totale de telle sorte à apporter 3375 BGU/animal/jour. Les caractéristiques du prémix ainsi standardisé sont : 70 AXC/g, 213 BGU/g et 28 CMC/g.

Les fermentations *in vitro* ont été réalisées en suivant la méthode décrite par Menke et Steingass (1988). L'inoculum était constitué pour 1/3 de jus de rumen collecté sur 2 vaches canulées et 2/3 de tampon de Menke et Steingass (Menke K H, Steingass H. (1988) Estimation of the energetic feed value obtained from chemical analysis and in vitro gas production using rumen fluid. Animal Research Development. 28, 7-55). 200 mg de substrat ont été incorporés dans des seringues en verre de 100 ml en présence de l'inoculum. La lecture du volume de gaz était réalisée régulièrement.

Le jus de rumen a été prélevé sur les animaux soumis au régime alimentaire normal (Control), puis sur les mêmes animaux après 3 semaines d'adaptation à un régime complémenté en composition enzymatique selon l'invention (Essai) à hauteur de 1 g/kg d'aliment (∼15 g/j/animal).

Le schéma expérimental était le suivant : 4 substrats × 2 traitements (Control ou Essai) × 3 seringues + 3 blancs = 27 seringues par série. Deux séries ont été réalisées (soit 54 seringues) pour la détermination de la production de méthane (CH₄) et de l'azote ammoniacale (N-NH₃).

Les fermentations des séries consacrées à la production de CH₄ et de N-NH3 ont été réalisées durant 24h. Au terme des 24h, un échantillon de la phase gazeuse des bouteilles de fermentation a été prélevé pour déterminer la production de CH₄ par chromatographie gazeuse et un échantillon de la phase liquide pour déterminer la production de N-NH3. Lors du démarrage des fermentations, 3 échantillons de l'inoculum ont été également centrifugés et analysés pour leur teneur en N-NH3.

Les productions de CH₄ ont été rapportées au g de MS de substrat mis en fermentation et les valeurs corrigées par rapport aux inoculums et aux blancs.

### Résultats

Du point de vue de la quantité de méthane produite après 24 h de fermentation, il semble apparaître une tendance à une production de méthane moindre avec la composition enzymatique selon l'invention pour les ingrédients fourragers (foin, ensilage préfané et ensilage de maïs) et une utilisation supérieure de l'ammoniac par le microbiote ruminai (teneur plus faible en NH3), comme le montre le Tableau 11.

**Tableau 11 : Production de méthane et d'ammonium après fermentation in vitro pendant 24 h d'aliments par du fluide ruminai en présence ou non de la composition enzymatique selon l'invention (N = 6)**

| | | CH₄ (mmol/g) | évolution / control (%) | NH₃ (mg/g) | évolution / control (%) |
|---|---|---|---|---|---|
| Foin | control | 1.779 | | 27.50 | |
| | Essai | 1.623 | -8.8 | 26.90 | -2.2 |
| Ensilage Maïs | control | 2.293 | | 21.40 | |
| | Essai | 2.274 | -0.8 | 20.70 | -3.3 |
| Ensilage Herbe | control | 1.428 | | 28.90 | |
| | Essai | 1.391 | -2.6 | 25.80 | -10.7 |
| Tourteau de soja | control | 2.315 | | 67.70 | |
| | Essai | 2.513 | 8.6 | 68.00 | 0.4 |
| SEM | | 0.075 | | 2.90 | |

Lors des mesures réalisées après 24 h (méthane et NH3), les inventeurs ont pu observer une tendance cohérente pour tous les aliments fibreux (fourrages : foin, ensilage de maïs et ensilage d'herbe préfanée) vers une réduction de la production de méthane et d'un usage plus important de l'ammoniac du tampon de fermentation par les micro-organismes du rumen. Ceci signifie que dans les premières heures de fermentation, les fourrages incubés en présence de la composition enzymatique selon l'invention pourraient favoriser une meilleure croissance bactérienne et dès lors assurer un meilleur approvisionnement en protéines et acides aminés d'origine microbienne aux vaches.

Cet exemple démontre que l'apport de composition enzymatique selon l'invention permet une réduction de la production de méthane par les micro-organismes contenus dans le rumen des ruminants.

### Exemple 6 : Mesure de l'effet de la composition enzymatique selon l'invention sur les digestibilités in sacco d'aliments pour ruminants

Dans cet exemple est décrit l'effet de la composition enzymatique selon l'invention sur la dégradabilité *in sacco* de différentes matières premières courantes dans l'alimentation des ruminants.

### Matériels et méthodes

### Animaux

Deux vaches réformées canulées au rumen ont été utilisées. Pendant l'entièreté de la durée de l'expérience *in sacco,* les animaux ont été nourris avec un mélange d'herbe préfanée ensilée (4 kg), d'ensilage de maïs (5 kg/j), paille hachée (1 kg) et de concentré (1kg).

### Ingrédients

Quatre aliments ont été étudiés *in sacco* : Foin d'herbe, Ensilage d'herbe, Ensilage de maïs et Tourteau de soja. La composition chimique des ingrédients expérimentaux est présentée dans le Tableau 12.

**Tableau 12 : Composition chimique des ingrédients utilisés**

| Ingrédients | MAT¹ (%) | NDF(%) | Amidon(%) | Energie brute (kcal/kg MS) |
|---|---|---|---|---|
| Soja | 55,37 | 15,18 | N/A² | 4636,92 |
| Préfané | 17,90 | 44,72 | N/A | 4106,45 |
| Maïs | 5,07 | 53,53 | 19,5 | 4546,62 |
| Foin | 6,89 | 65,93 | N/A | 4306,68 |

| | | | | |
|---|---|---|---|---|
| ¹MAT: Matière azote totale ²N/A : Non applicable | | | | |

Les différents ingrédients ont été lyophilisés et moulus à 1 mm au moyen d'un moulin de laboratoire.

La composition enzymatique, préparée selon le procédé de production décrit dans l'exemple 1 en utilisant une souche d'*Aspergillus neoniger,* a été standardisée sous forme d'un prémix pour assurer une bonne homogénéité de la composition et faciliter son utilisation lors de l'étape de distribution aux animaux en « top feeding ». Le prémix obtenu a été mélangé dans la ration totale de telle sorte à apporter 3375 BGU/animal/jour. Les caractéristiques du prémix ainsi standardisé sont : 70 AXC/g, 213 BGU/g et 28 CMC/g.

Pour étudier les changements induits par la composition enzymatique selon l'invention sur le métabolisme ruminai chez l'animal, les 2 vaches laitières taries canulées ont été utilisées pour réaliser une expérience *in sacco* avec utilisation de la technique des sachets nylon (Ørskov, E. R., Hovell, D and Mould, F. L. (1980). The use of the nylon bag technique for the evaluation of feedstuffs. Tropical Animal Production 5: 195-213.). Deux séries de digestibilités *in sacco* ont été réalisées sur les quatre aliments testés : une série avec les animaux soumis au régime alimentaire décrit ci-dessus (Control), suivie d'une série réalisée sur les mêmes animaux après 3 semaines d'adaptation à un régime complémenté (Essai) en composition enzymatique selon l'invention à hauteur de environ 1 g/kg d'aliment (∼15 g/j/animal). Le schéma expérimental était le suivant : 4 ingrédients × 2 traitements (Control ou Essai) × 4 sachets × 7 temps de mesure (0, 3, 6, 9, 12, 24, 48 h) = 224 sacs. Les aliments fermentés dans les sacs (0, 6, 12, 24, 48 h) ont été analysés pour leurs teneurs en protéines brutes, NDF, énergie et amidon (amidon uniquement pour l'ensilage de maïs).

Les teneurs en matière sèche, et protéines brutes des ingrédients avant et après hydrolyse ont été déterminées via les méthodes de références (méthode 967.03; méthode 981.10 ; "Official methods of analysis", AOAC, 1990, Ed. Kenneth Helrich). Les teneurs en amidon de l'ensilage de maïs ont été déterminées par une méthode enzymatique et colorimétrique (Megazyme Ltd, Ireland). Les teneurs en NDF (Neutral Detergent Fiber) suivant la méthode de Van Soest *et al.* (Van Soest P.J., Robertson J.B., Lewis B.A. (1991): Methods for dietary fiber, neutral detergent fiber, and nonstarch polysaccharides in relation to animal nutrition. J. Dairy Sci., 74, 3583-3597) ont été réalisées en utilisant de l'amylase thermostable avec le système ANKOM.

Au terme des digestions *in sacco,* les digestibilités de la matière sèche, des protéines, de l'énergie, du NDF et de l'amidon ont été calculées et les valeurs comparées statistiquement suivant l'ingrédient considéré et la complémentation ou non (N = 8) et la cinétique de dégradation modélisée au moyen du modèle d'Orskov et McDonald (Orskov, E.R. and McDonald, Y. 1979. The estimation of protein degradability in the rumen from determining the digestibility of feeds in the rumen. Journal Agricultural Science, Cambridge 92:499-503.). La vache a été considérée comme un facteur aléatoire.

### Résultats

La dégradabilité finale de la matière sèche des ingrédients contenus dans les sachets nylon incubés dans le rumen des vaches canulées n'a pas été significativement altérée par la distribution de la composition enzymatique selon l'invention aux vaches (Tableau 13). Cependant, la cinétique de dégradation a bien été influencée pour les fourrages et en particulier en ce qui concerne l'ensilage d'herbe préfanée puisque la dégradabilité après 24 h atteignait 0,820 en présence de la composition enzymatique selon l'invention contre 0,645 sans (P<0,05).

Cette tendance s'est confirmée dans une dégradation plus rapide de la fraction de fibres non solubles dans le détergent neutre (NDF) (Tableau 14). En effet, l'addition de la composition enzymatique selon l'invention fait passer la dégradabilité du NDF après 24h de 0,370 à 0,685 pour l'herbe préfanée (P<0,05), et la même tendance est observée pour le maïs et le foin, ainsi que l'azote (Tableau 15) avec des différences toutefois non significatives (P>0,05).

La dégradabilité de l'énergie brute contenue dans le soja après 6 et 12 h était supérieure quand les vaches étaient complémentées avec la composition enzymatique selon l'invention par rapport aux vaches non-complémentées (0,600 et 0,540 vs. 0,420 et 0,490) (Tableau 16).

**Tableau 13 : Digestibilité de la matière sèche lors de l'in sacco en présence ou non de la composition enzymatique selon l'invention (N = 4) (g/g aliment) après 0, 3, 6, 9, 12, 24 et 48h**

| | | **Digestibilité de la matière sèche (g/g aliment, n=4)** | | | | | | |
|---|---|---|---|---|---|---|---|---|
| Tps fermentation (h) | | 0 | 3 | 6 | 9 | 12 | 24 | 48 |
| Soja | Essai | 0.409 | 0.480 | 0.625 | 0.695 | 0.715 | 0.880 | 0.968 |
| | Control | 0.409 | 0.496 | 0.480 | 0.651 | 0.735 | 0.860 | 1.001 |
| Ensilage Herbe | Essai | 0.409 | 0.424 | 0.495 | 0.551 | 0.560 | **0.820** | 0.855 |
| | Control | 0.409 | 0.444 | 0.470 | 0.540 | 0.590 | **0.645** | 0.845 |
| Ensilage Maïs | Essai | 0.407 | 0.477 | 0.515 | 0.489 | 0.605 | 0.650 | 0.760 |
| | Control | 0.407 | 0.474 | 0.480 | 0.534 | 0.545 | 0.585 | 0.735 |
| Foin | Essai | 0.235 | 0.242 | 0.270 | 0.307 | 0.320 | 0.445 | 0.570 |
| | Control | 0.235 | 0.263 | 0.290 | 0.316 | 0.315 | 0.390 | 0.550 |

| | | | | | | | | |
|---|---|---|---|---|---|---|---|---|
| **en gras** : différence significative (p<0.05) | | | | | | | | |

**Tableau 14 : Digestibilité de NDF de l'in sacco en présence ou non de la composition enzymatique selon l'invention (N = 2) (g/g aliment) après 0, 6, 12, 24 et 48 h.**

| | | **Digestibilité du NDF (g/g aliment, n=2)** | | | | |
|---|---|---|---|---|---|---|
| Tps fermentation (h) | | 0 | 6 | 12 | 24 | 48 |
| Soja | Essai | 0.242 | 0.390 | 0.480 | * | * |
| | Control | 0.240 | 0.270 | 0.605 | * | * |
| Ensilage Herbe | Essai | 0.028 | 0.080 | 0.270 | **0.685** | 0.765 |
| | Control | 0.028 | 0.060 | 0.200 | **0.370** | 0.730 |
| Ensilage Maïs | Essai | 0.072 | 0.225 | 0.360 | 0.440 | 0.620 |
| | Control | 0.072 | 0.180 | 0.285 | 0.335 | 0.585 |
| Foin | Essai | 0.028 | 0.045 | 0.120 | 0.285 | 0.455 |
| | Control | 0.028 | 0.100 | 0.125 | 0.220 | 0.430 |

| | | | | | | |
|---|---|---|---|---|---|---|
| * : pas assez d'échantillon dans le sachet nylon pour effectuer le dosage **en gras** : différence significative (p<0.05) | | | | | | |

**Tableau 15 : Digestibilité de l'azote de l'in sacco en présence ou non de la composition enzymatique selon l'invention (N = 2) (g/g aliment) après 0, 6, 12, 24 et 48 h.**

| | **Digestibilité de MAT (g/g aliment, n=2)** | | | | | |
|---|---|---|---|---|---|---|
| Tps fermentation (h) | | 0 | 6 | 12 | 24 | 48 |
| Soja | Essai | 0.228 | 0.590 | 0.595 | * | * |
| | Control | 0.228 | 0.325 | 0.625 | * | * |
| Ensilage Herbe | Essai | 0.303 | 0.665 | 0.720 | 0.910 | 0.955 |
| | Control | 0.303 | 0.640 | 0.740 | 0.785 | 0.935 |
| Ensilage Maïs | Essai | 0.705 | 0.705 | 0.745 | 0.765 | 0.780 |
| | Control | 0.705 | 0.715 | 0.705 | 0.745 | 0.780 |
| Foin | Essai | 0.303 | 0.420 | 0.395 | 0.525 | 0.655 |
| | Control | 0.303 | 0.375 | 0.415 | 0.475 | 0.655 |

| | | | | | | |
|---|---|---|---|---|---|---|
| * : pas assez d'échantillon dans le sachet nylon pour effectuer le dosage **en gras** : différence significative (p<0.05) | | | | | | |

**Tableau 16 : Digestibilité de l'énergie brute in sacco en présence ou non de la composition enzymatique selon l'invention (N = 2) (g/g aliment) après 0, 6, 12, 24 et 48 h.**

| | **Digestibilité de l'énergie brute (g/g aliment, n=2)** | | | | | |
|---|---|---|---|---|---|---|
| Tps fermentation (h) | | 0 | 6 | 12 | 24 | 48 |
| Soja | Essai | 0.374 | **0.600** | **0.540** | * | * |
| | Control | 0.374 | **0.420** | **0.490** | * | * |
| Ensilage Herbe | Essai | 0.199 | 0.435 | 0.660 | 0.806 | 0.838 |
| | Control | 0.199 | 0.415 | 0.505 | 0.678 | 0.827 |
| Ensilage Maïs | Essai | 0.414 | 0.520 | 0.530 | 0.570 | 0.760 |
| | Control | 0.414 | 0.490 | 0.550 | 0.590 | 0.680 |
| Foin | Essai | 0.199 | 0.240 | 0.285 | 0.415 | 0.490 |
| | Control | 0.199 | 0.220 | 0.285 | 0.350 | 0.515 |

| | | | | | | |
|---|---|---|---|---|---|---|
| * : pas assez d'échantillon dans le sachet nylon pour effectuer le dosage **en gras** : différence significative (p<0.05) | | | | | | |

L'hypothèse d'un impact important de la composition enzymatique selon l'invention sur les aliments fibreux est confirmée avec ces mesures de dégradabilité *in sacco.* La composition enzymatique selon l'invention accélère les processus de dégradation des 3 aliments fibreux et d'une manière significative elle permet d'accélérer la fermentation de l'ensilage d'herbe préfanée et particulièrement sa fraction fibreuse (NDF). Les conséquences chez les vaches laitières pourraient en être une réduction de l'encombrement ruminai et une augmentation de la capacité d'ingestion des fourrages. Cet effet serait particulièrement intéressant pour des animaux en lactation qui éprouvent des difficultés à satisfaire leurs besoins alimentaires en consommant des fourrages et qui dès lors doivent être complémentés d'une manière importante par des concentrés de productions plus coûteux que les ressources fourragères.

Cet exemple démontre que l'apport de composition enzymatique selon l'invention permet une meilleure valorisation des ressources fourragères par les animaux et donc une meilleure efficacité alimentaire.

### Exemple 7 : Mesure de l'effet de la composition enzymatique selon l'invention sur le métabolisme des microbes du rumen en réacteur anaérobie à trop-plein (RATs) et en tubes de culture.

Les objectifs de cet exemple sont de :
1) Quantifier les effets de la composition enzymatique selon l'invention sur le métabolisme de microbes du rumen bovin cultivés dans 6 réacteurs anaérobies à trop-plein (RAT) sur trois rations caractéristiques de l'élevage bovin. Les RATs sont des fermenteurs continus à double effluent.
2) Préciser le mode d'action de la composition enzymatique selon l'invention sur les fermentations ruminales, en mesurant, en tubes de culture, l'impact de la composition enzymatique brute, de ses fractions soluble et insoluble et de la composition enzymatique inactivée par autoclavage sur les productions d'acides gras volatils (AGV) et de gaz au bout de 24h d'incubation.

### Matériels et méthodes

### Essai en réacteur anaérobie à trop-plein (RAT)

Sont utilisés six fermenteurs de 1 litre de volume de travail, réglés aux taux de renouvellement de la phase solide et de la phase liquide de 0.03 et 0.06 h⁻¹ respectivement. Ces fermenteurs sont inoculés par du contenu de rumen de vache laitière filtré sur toile de 1 mm. Le pH des fermenteurs est maintenu au-dessus de 6.0 par infusion continue de solutions tampon (carbonate et phosphate). L'apport de substrats (aliments) s'effectue à heure fixe: à 11h et 23h. Une phase d'équilibration des fermenteurs s'effectue pendant les 5 premiers jours avant la phase de mesures et prélèvements.

La composition enzymatique selon l'invention est introduite à un niveau de 10 g par kg de MS ration. L'apport journalier est de 0,25 g par fermenteur. Deux lots de composition enzymatique sont préparés selon le procédé de production décrit dans l'exemple 1 en utilisant, d'une part, une souche *d'Aspergillus tubingensis* (n°1) et, d'autre part, une souche *d'Aspergillus neoniger* (n°2). Les compositions enzymatiques ainsi obtenues présentent les caractéristiques suivantes : pour la version n°1 1037 AXC/g, 1063 BGU/g et 262 CMC/g ; pour la version n°2 3399 AXC/g, 3623 BGU/g et 846 CMC/g. Les deux compositions obtenues ont été standardisées sous forme de deux prémix afin de faciliter son incorporation dans les fermenteurs. Les caractéristiques des prémix ainsi standardisés sont : 171 AXC/g, 225 BGU/g et 67 CMC/g pour la version n°1 ; 122 AXC/g, 280 BGU/g et 75 CMC/g pour la version n°2.

Les rations contiennent du blé et un tourteau de soja de façon à formuler des régimes iso-azotés et iso-énergétiques.

Les différents facteurs testés sont :
1) Produit, facteur à 3 niveaux : absence / composition enzymatique n°1 / composition enzymatique n°2
2) Régimes, facteur à 3 niveaux : à base d'ensilage de maïs / d'ensilage d'herbe / de foin de dactyle

Le plan d'expérience choisi est un plan en 3 blocs incomplets équilibrés (Tableau 17) correspondant à 3 périodes expérimentales de 7 jours, chacune comprenant une phase d'équilibration de 5 jours et une phase de mesures et de prélèvements de 2 jours.

**Tableau 17 : Plan d'expérience des essais en RATs avec les deux versions de composition enzymatique et les différents aliments.**

| | | fermenteurs | | | | | |
|---|---|---|---|---|---|---|---|
| | | A | B | C | D | E | F |
| bloc 1 | régime | EH | EM | F | F | EM | EH |
| | Composition enzymatique | N°1 | N°1 | 0 | N°2 | 0 | N°2 |
| bloc 2 | régime | F | EH | EH | EM | F | EM |
| | Composition enzymatique | N°2 | 0 | N°1 | N°2 | N°1 | 0 |
| bloc 3 | régime | EH | EM | EM | F | EH | F |
| | Composition | 0 | N°1 | N°2 | 0 | N°2 | N°1 |
| enzymatique | | | | | | | |

| | | | | | | | |
|---|---|---|---|---|---|---|---|
| EH : ensilage d'herbe ; EM : ensilage de maïs ; F : foin de prairie | | | | | | | |

Les mesures et prélèvements effectués au cours des essais sont :
- pH, potentiel redox.
- Contenu de fermenteur pour dénombrement des protozoaires et détermination du faciès fermentaire (AGV, azote ammoniacal).
- Gaz (collecte pendant 2 jours) pour mesure du volume produit et de sa composition
- Effluents collectés durant deux jours, pour bilans de dégradation des rations, fermentation et synthèse de protéines microbiennes.
- Culots microbiens par double centrifugation des effluents frais.

Les analyses suivantes sont réalisées sur les prélèvements :
- AGV (chromatographie) dans les ensilages, les effluents et les contenus de fermenteur
- Azote ammoniacal (sonde spécifique)
- Gaz (chromatographie)
- MS, matière organique dans les aliments et les effluents lyophilisés
- Amidon dans les aliments et les effluents lyophilisés
- Fractionnement des parois selon Van Soest (NDF, ADF, ADL) dans les aliments et les effluents lyophilisés
- Azote (méthode Dumas) dans les aliments, les effluents lyophilisés et les culots microbiens.
- Bases nucléiques (chromatographie) dans les effluents lyophilisés et les culots microbiens.

Une analyse statistique est réalisée avec un modèle d'analyse de variance comme suit : Yᵢⱼₖₗ = Pᵢ + Rⱼ + Pᵢ*Rⱼ + Bₖ + eᵢⱼₖₗ avec P, R, B et e étant respectivement les facteurs Produit et Régime, le bloc et l'erreur expérimentale.

### Essai en tubes de culture

Sont inoculés 60 tubes de culture de 72 mL, équipés de bouchons avec septum butyl-teflon, avec du contenu de fermenteur prélevé 12h après le dernier apport de ration et incubés pendant 24 h selon la procédure standard. Les facteurs étudiés sont :
1) Produit, facteur P à 5 niveaux : absence / Composition n°2 / fraction insoluble de la Composition n°2 / fraction liquide de la Composition n°2 (dépourvue du substrat de fermentation) / Composition enzymatique inactivée par autoclavage. La fraction liquide de la Composition n°2 correspond aux enzymes extraites en phase aqueuse du reste de la Composition n°2.
2) Inoculum, facteur R à 3 niveaux : adapté à un régime à base d'ensilage de maïs / d'ensilage d'herbe / de foin de dactyle et recevant le même régime en tubes de culture.

La composition enzymatique selon l'invention n°2 est introduite avec la ration à un niveau de 10 g par kg de MS ration.

Le plan d'expérience est organisé en 2 périodes de 2 jours consécutifs pour chaque inoculum. Les inoculums sont prélevés sur les RATs ne recevant pas de composition enzymatique (niveau 0 du facteur Produit dans le plan d'expérience en RATs) après la période d'équilibration. Deux tubes de culture sont lancés par traitement (combinaison Produit x Inoculum) et par jour d'incubation.

Les mesures et prélèvements effectués au cours des essais sont :
- Inoculum pour détermination de sa composition en AGV
- Milieu de culture après 24 h d'incubation pour tubes pour détermination de la composition en AGV.
- Gaz pour mesure de la quantité produite après 24 h d'incubation et détermination de sa composition

Les analyses suivantes sont réalisées sur les prélèvements :
- AGV (chromatographie) dans les ensilages, les effluents et les contenus de fermenteur
- Gaz (chromatographie)

Une analyse statistique est réalisée avec un modèle d'analyse de variance.

Cette analyse montre l'effet bénéfique de la composition enzymatique selon l'invention sur le métabolisme des microbes du rumen, par rapport à l'effet des enzymes isolées du substrat de fermentation sur ce métabolisme.

### Exemple 8 : Production d'une composition enzymatique selon l'invention par fermentation en milieu solide

Dans cet exemple est décrite une méthode d'obtention de la composition enzymatique selon l'invention par fermentation en milieu solide d'un substrat constitué d'un mélange tourteau de colza et son de blé (substrat principal) et de tourteau de germes de maïs (substrat complémentaire) en présence d'une souche d'*Aspergillus tubingensis.*

Un milieu nutritif est constitué par un mélange de tourteau de colza en farine et de son de blé complété avec un tourteau de germes de maïs, dans la proportion en poids suivante : 36% de tourteau de colza, 36% de son de blé et 28% de tourteau de germes de maïs. Le mélange est ensuite pré-humidifié à 60% de matière sèche et autoclavé pendant 35 min à 105°C. Après refroidissement, le milieu est inoculé par une solution de spores d'*Aspergillus tubingensis* afin d'obtenir une concentration de 1 × 10⁷ spores par gramme de matière sèche et une humidité initiale de 45%. Le pH est ajusté à 4,9 par ajout d'acide sulfurique. Le milieu de culture ainsi obtenu est réparti dans des fioles d'erlenmeyer à raison de 10 g de matière sèche par fiole. Les fioles d'erlenmeyer sont ensuite incubées à 33°C en conditions aérobies à l'obscurité pendant 72 h sans agitation. La culture est arrêtée à l'apparition des premières spores dans le milieu de culture (la présence de spores pouvant gêner les animaux lors de l'ingestion du fait de leur caractère volatil, les produits fermentés sont conçus de manière à en contenir le moins possible).

Le mélange obtenu en fin de fermentation peut être séché et utilisé tel quel dans l'alimentation des animaux ou être extrait dans l'eau pour être ajouté sous forme liquide.

Les caractéristiques de la composition ainsi obtenue après 72 h de fermentation sont : 2650 AXC/g, 4808 BGU/g et 1050 CMC/g.

## Revendications

1. Composition enzymatique comprenant un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, fermenté avec une souche d'*Aspergillus* section *Nigri* du sous-clade *Aspergillus tubingensis* par fermentation en milieu solide, ladite composition enzymatique présentant:
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition.

2. Composition enzymatique selon la revendication 1, dans laquelle la souche d'*Aspergillus* section *Nigri* est une souche d'*Aspergillus tubingensis* ou d'*Aspergillus neoniger.*

3. Composition enzymatique selon l'une des revendications 1 à 2, dans laquelle la souche d'*Aspergillus* section *Nigri* est une souche d'*Aspergillus tubingensis.*

4. Composition enzymatique selon l'une quelconque des revendications 1 à 3, présentant:
(i) une activité xylanase supérieure ou égale à 1000 AXC par gramme de composition,
(ii) une activité β-glucanase supérieure ou égale à 800 BGU par gramme de composition, et
(iii) une activité cellulase supérieure ou égale à 120 CMC par gramme de composition.

5. Procédé de fabrication d'une composition enzymatique telle que définie dans la revendication 1, comprenant une étape de fermentation en milieu solide d'un substrat principal, sélectionné dans le groupe constitué d'un blé, un son de blé, un tourteau de colza, un mélange d'un blé et d'un son de blé, un mélange d'un blé et d'un tourteau de colza, un mélange d'un son de blé et d'un tourteau de colza, et un mélange d'un blé, d'un son de blé et d'un tourteau de colza, avec une souche du sous-clade *A. tubingensis,* au moins jusqu'à ce que le produit de fermentation présente les valeurs minimales suivantes d'activité enzymatique :
(i) une activité xylanase supérieure ou égale à 500 AXC par gramme de composition,
(ii) une activité β-glucanase supérieure ou égale à 500 BGU par gramme de composition, et
(iii) une activité cellulase supérieure ou égale à 50 CMC par gramme de composition.

6. Procédé de de fabrication selon la revendication 5, dans lequel la souche du sous-clade *Aspergillus tubingensis* est une souche d'*Aspergillus tubingensis.*

7. Additif pour l'alimentation des ruminants qui comprend la composition enzymatique selon l'une quelconque des revendications 1 à 4.

8. Utilisation de l'additif selon la revendication 7 comme ingrédient de compositions alimentaires ou de prémix pour l'alimentation des ruminants.

9. Prémix pour l'alimentation des ruminants comprenant un additif selon la revendication 7.

10. Composition alimentaire supplémentée pour ruminants comprenant une quantité efficace d'un additif selon la revendication 7 ou d'un prémix selon la revendication 9, en association avec des aliments appropriés pour les ruminants.

11. Procédé de fabrication d'une composition alimentaire supplémentée pour ruminants comprenant le mélange d'un additif selon la revendication 7 ou d'un prémix selon la revendication 9 avec des aliments appropriés pour les ruminants.

12. Utilisation non-thérapeutique d'une composition enzymatique selon l'une quelconque des revendications 1 à 4, d'un additif selon la revendication 7, d'un prémix selon la revendication 9 ou d'une composition alimentaire selon la revendication 10, pour améliorer les performances zootechniques d'un ruminant.

13. Procédé non-thérapeutique d'amélioration des performances zootechniques d'un ruminant dans lequel on fait ingérer au ruminant une quantité efficace de la composition enzymatique selon l'une quelconque des revendications 1 à 4, de l'additif selon la revendication 7, du prémix selon la revendication 9 ou de la composition alimentaire selon la revendication 10.

## Patentansprüche

1. Enzymatische Zusammensetzung, die ein Hauptsubstrat enthält, ausgewählt aus der Gruppe, bestehend aus Weizen, Weizenkleie, Rapsschrot, einer Mischung aus Weizen und Weizenkleie, einer Mischung aus Weizen und Rapsschrot, einer Mischung aus Weizenkleie und Rapsschrot und einer Mischung aus Weizen, Weizenkleie und Rapsschrot, fermentiert mit einem Stamm des Aspergillus Sektion Nigri der Unterart Aspergillus tubingensis durch Feststoffgärung, wobei die enzymatische Zusammensetzung aufweist:
(i) eine Xylanase-Aktivität größer als oder gleich 500 AXC pro Gramm der Zusammensetzung,
(ii) eine β-Glucanase-Aktivität größer als oder gleich 500 BGU pro Gramm der Zusammensetzung,
(iii) eine Zellulase-Aktivität größer als oder gleich 50 CMC pro Gramm der Zusammensetzung.

2. Enzymatische Zusammensetzung nach Anspruch 1, bei der der Stamm des Aspergillus Sektion Nigri ein Stamm Aspergillus tubingensis oder Aspergillus neoniger ist.

3. Enzymatische Zusammensetzung nach Anspruch 1 bis 2, bei der der Stamm des Aspergillus Sektion Nigri ein Stamm Aspergillus tubingensis ist.

4. Enzymatische Zusammensetzung nach einem beliebigen der Ansprüche 1 bis 3, aufweisend:
(i) eine Xylanase-Aktivität größer als oder gleich 1000 AXC pro Gramm der Zusammensetzung,
(ii) eine β-Glucanase-Aktivität größer als oder gleich 800 BGU pro Gramm der Zusammensetzung,
(iii) eine Zellulase-Aktivität größer als oder gleich 120 CMC pro Gramm der Zusammensetzung.

5. Verfahren zur Herstellung einer enzymatische Zusammensetzung, wie im Anspruch 1 definiert, das einen Schritt der Feststoffgärung eines Hauptsubstrats umfasst, das ausgewählt ist aus der Gruppe, bestehend aus Weizen, Weizenkleie, Rapsschrot, einer Mischung aus Weizen und Weizenkleie, einer Mischung aus Weizen und Rapsschrot, einer Mischung aus Weizenkleie und Rapsschrot und einer Mischung aus Weizen, Weizenkleie und Rapsschrot, mit einem Stamm der Unterart Aspergillus tubingensis zumindest bis das Gärungsprodukt die folgenden minimalen Werte der enzymatischen Aktivität aufweist:
(i) eine Xylanase-Aktivität größer als oder gleich 500 AXC pro Gramm der Zusammensetzung,
(ii) eine β-Glucanase-Aktivität größer als oder gleich 500 BGU pro Gramm der Zusammensetzung,
(iii) eine Zellulase-Aktivität größer als oder gleich 50 CMC pro Gramm der Zusammensetzung.

6. Herstellungsverfahren nach Anspruch 5, bei dem der Stamm der Unterart Aspergillus tubingensis ein Stamm Aspergillus tubingensis ist.

7. Zusatz für die Ernährung von Wiederkäuern, der die enzymatische Zusammensetzung nach einem beliebigen der Ansprüche 1 bis 4 enthält.

8. Verwendung des Zusatzes nach Anspruch 7 als Zutat von Futterzusammensetzungen oder einer Vormischung für die Ernährung von Wiederkäuern.

9. Vormischung für die Ernährung von Wiederkäuern, die einen Zusatz nach Anspruch 7 enthält.

10. Angereicherte Nahrungszusammensetzung für Wiederkäuer, die eine wirksame Menge eines Zusatzes nach Anspruch 7 oder eine Vormischung nach Anspruch 9 zusammen mit geeigneten Futtermitteln für die Wiederkäuer enthält.

11. Verfahren zur Herstellung einer angereicherten Nahrungszusammensetzung für Wiederkäuer, das eine Mischung eines Zusatzes nach Anspruch 7 oder eine Vormischung nach Anspruch 9 mit geeigneten Futtermitteln für Wiederkäuer umfasst.

12. Nichttherapeutische Verwendung einer enzymatische Zusammensetzung nach einem beliebigen der Ansprüche 1 bis 4, eines Zusatzes nach Anspruch 7, einer Vormischung nach Anspruch 9 oder einer Nahrungszusammensetzung nach Anspruch 10 zum Verbessern der zuchttechnischen Leistungen eines Wiederkäuers.

13. Nichttherapeutisches Verfahren zum Verbessern der zuchttechnischen Leistungen eines Wiederkäuers, bei dem eine wirksame Menge der enzymatischen Zusammensetzung nach einem beliebigen der Ansprüche 1 bis 4, des Zusatzes nach Anspruch 7, der Vormischung nach Anspruch 9 oder der Nahrungszusammensetzung nach Anspruch 10 einem Wiederkäuer eingegeben wird.

## Claims

1. An enzymatic composition comprising a main substrate, selected from the group consisting of wheat, wheat bran, rapeseed cake, a mixture of wheat and wheat bran, a mixture of wheat and rapeseed cake, a mixture of wheat bran and rapeseed cake, and a mixture of wheat, wheat bran and a rapeseed cake, fermented with a strain of *Aspergillus* section *Nigri* of the sub-clade *Aspergillus tubingensis* by solid-state fermentation, said enzymatic composition displaying:
(i) a xylanase activity greater than or equal to 500 AXC per gram of composition,
(ii) a β-glucanase activity greater than or equal to 500 BGU per gram of composition, and
(iii) a cellulase activity greater than or equal to 50 CMC per gram of composition.

2. The enzymatic composition according to claim 1, wherein the strain of *Aspergillus* section *Nigri* is a strain of *Aspergillus tubingensis* or *Aspergillus neoniger.*

3. The enzymatic composition according to claim 1 or 2, wherein the strain of *Aspergillus* section *Nigri* is a strain of *Aspergillus tubingensis.*

4. The enzymatic composition according to any one of claims 1 to 3, displaying:
(i) a xylanase activity greater than or equal to 1000 AXC per gram of composition,
(ii) a β-glucanase activity greater than or equal to 800 BGU per gram of composition, and
(iii) a cellulase activity greater than or equal to 120 CMC per gram of composition.

5. A method for manufacturing an enzymatic composition as defined in claim 1, comprising a step of solid-state fermentation of a main substrate, selected from the group consisting of wheat, wheat bran, rapeseed cake, a mixture of wheat and wheat bran, a mixture of wheat and rapeseed cake, a mixture of wheat bran and rapeseed cake, and a mixture of wheat, wheat bran and rapeseed cake, with a strain of the sub-clade *A*. *tubingensis,* at least until the fermentation product displays the following minimum values of enzymatic activity :
(i) a xylanase activity greater than or equal to 500 AXC per gram of composition,
(ii) a β-glucanase activity greater than or equal to 500 BGU per gram of composition, and
(iii) a cellulase activity greater than or equal to 50 CMC per gram of composition.

6. The method for manufacturing according to claim 5, wherein the strain of the sub-clade *Aspergillus tubingensis* is a strain of *Aspergillus tubingensis.*

7. An additive for the feed of ruminants which comprises the enzymatic composition according to any one of claims 1 to 4.

8. Use of the additive according to claim 7 as an ingredient of feed compositions or of premixes for feeding ruminants.

9. A premix for the feed of ruminants comprising an additive according to claim 7.

10. A supplemented feed composition for ruminants comprising an effective amount of an additive according to claim 7 or of a premix according to claim 9, in association with feedstuffs suitable for ruminants.

11. A method for manufacturing a supplemented feed composition for ruminants comprising mixing an additive according to claim 7 or a premix according to claim 9 with feedstuffs suitable for ruminants.

12. Non-therapeutic use of an enzymatic composition according to any one of claims 1 to 4, of an additive according to claim 7, of a premix according to claim 9 or of a feed composition according to claim 10, for improving the zootechnical performances of a ruminant.

13. A non-therapeutic method for improving zootechnical performances of a ruminant in which the ruminant is made to ingest an effective amount of the enzymatic composition according to any one of claims 1 to 4, of the additive according to claim 7, of the premix according to claim 9 or of the feed composition according to claim 10.
